(19) **Europäisches Patentamt / European Patent Office / Office européen des brevets**

(11) **EP 4 640 694 A1**

(12) # EUROPEAN PATENT APPLICATION
published in accordance with Art. 153(4) EPC

(43) Date of publication:
**29.10.2025   Bulletin 2025/44**

(21) Application number: **23906078.3**

(22) Date of filing: **22.12.2023**

(51) International Patent Classification (IPC):
**C07K 7/08** *(2006.01)*      **A61K 47/64** *(2017.01)*

(52) Cooperative Patent Classification (CPC):
**A61K 47/64; C07K 7/08**

(86) International application number:
**PCT/CN2023/140904**

(87) International publication number:
**WO 2024/131923 (27.06.2024 Gazette 2024/26)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:   **23.12.2022   CN 202211671861**

(71) Applicant: **CYTOPORT (TIANJIN)
BIOTECHNOLOGY CO., LTD
Tianjin 300380 (CN)**

(72) Inventors:
• **LIANG, Jun**
  **Tianjin 300380 (CN)**
• **XIA, Yating**
  **Tianjin 300380 (CN)**

(74) Representative: **Grünecker Patent- und
Rechtsanwälte
PartG mbB
Leopoldstraße 4
80802 München (DE)**

Remarks:
The complete document including Reference
Table(s) and the Sequence Listing(s) can be
downloaded from the EPO website

(54) ## SELF-ASSEMBLING PEPTIDE

(57)    The present invention relates to a self-assembling peptide, a hydrogel formed from the self-assembling peptide, and an application of the hydrogel.

EP 4 640 694 A1

**Description**

**FIELD OF THE INVENTION**

The present invention belongs to the field of biomedical materials and specifically relates to peptide hydrogel biomaterials.

**BACKGROUND OF THE INVENTION**

Self-assembling peptides have attracted attention due to their good biocompatibility and diverse functions. Self-assembling peptides have been found to spontaneously form aggregates with certain structures in response to a specific factor. For example, these self-assembling peptides can respond to different factors such as temperature, pH, light, metal ions, a specific protein, thus initiating self-assembling behavior and forming a network scaffold structure. As a result, they can exert functions such as cellular support, bacteriostasis, and tissue repair. They can be used for applications such as drug delivery, drug screening and evaluation, antibacterial, tissue engineering, and 3D cell culture. However, these response factors may also impose certain limitations on practical application and increase the downside risk of application. For example, higher or lower pH (as disclosed in CN201680012178.5, the peptide hydrogel must be applied at pH < 3.5) and higher concentration of salt ions can cause cell damage (as disclosed in CN202110863352.X, the peptide hydrogel for promoting skin repair requires a specific concentration of sodium ions); the introduction of some exogenous initiator substances (as disclosed in CN201810783581.9, the peptide compound hydrogel needs the introduction of calcium phosphate for gelation) may lead to certain safety risks to a human body in clinical application; and temperature, light (as disclosed in CN201680061179.9, the gelation of peptides depends on light activation) and other responsive factors make the setting of self-assembly initiating conditions a factor that is necessary to be considered in practical applications and thus impose certain limitations on the application conditions of self-assembling polypeptides. When the above hydrogels are used in biological experiments, non-physiological conditions such as temperature and pH pose a non-negligible threat to cell activity; achieving light-responsive gelation determines on complex and expensive external devices; higher concentration of metal salt ions can affect the osmotic pressure of the cellular microenvironment; and exogenous substances may cause an immune reaction, thereby posing certain safety risks. However, currently available peptides capable of responding to endogenous substances to achieve gelation are limited in variety, and most are restricted to a single endogenous substance (for example, a peptide-albumin hydrogel disclosed in CN201380063880.0), which limits their scope of application.

Therefore, the development of self-assembling peptides that can respond and self-assemble into nanonetwork structures under a physiological condition, cell storage and culture environment, and in the presence of substances common in the human body, and that can exert support and repair functions even in a solution state, will have important application prospects.

**SUMMARY OF THE INVENTION**

The present invention relates to a self-assembling peptide, wherein the self-assembling peptide can respond and self-assemble into a nanonetwork structure under a wide variety of endogenous substances, and a self-assembling peptide solution system can exert support and repair functions even in a solution state.

In a first aspect, the present invention provides a self-assembling peptide that comprises a hydrophobic domain and a hydrophilic domain, wherein the hydrophilic domain comprises at least two consecutive β-turn regions capable of forming a β-turn.

In some embodiments, at least one β-turn region comprises or is linked to one or more acidic amino acids, preferably comprises or is linked to one acidic amino acid, at a terminus.

In some embodiments, at least one β-turn region comprises an acidic amino acid at the terminus.

In some embodiments, the β-turn region comprises a β-turn motif formed by 3-6 amino acids, and the β-turn motif has the following structure:

$X_1X_2X_3$, $X_1X_2X_3X_4$, $X_1X_2X_3X_4X_5$, or $X_1X_2X_3X_4X_5X_6$,
wherein $X_1$, $X_2$, $X_3$, $X_4$, $X_5$, and $X_6$ are each an amino acid residue, and $X_1$, $X_2$, $X_3$, $X_4$, $X_5$, and $X_6$ in each β-turn motif may be same or different from each other, respectively.

In some embodiments, the β-turn motif comprises a hydroxyproline (O), preferably, the $X_2$ is a hydroxyproline (O).

In some embodiments, the hydrophilic domain comprises 2-8 β-turn regions.

In some embodiments, the hydrophilic domain comprises 2, 3, 4, 5, 6, 7 or 8 β-turn regions.

Preferably, the hydrophilic domain comprises 2-6 β-turn regions. More preferably, the hydrophilic domain comprises 2-4 β-turn regions.

Preferably, the hydrophilic domain comprises 2 or 3 β-turn regions.

In some embodiments, the β-turn motif in at least the one β-turn region comprises or is linked to an acidic amino acid.

In some embodiments, the β-turn motif in at least the one β-turn region comprises or is linked to a glutamic acid (E), a valine (V), a leucine (L), an isoleucine (I), an aspartic acid (D), or a lysine (K).

In some embodiments, the hydrophilic domain comprises two β-turn regions, and the β-turn regions comprises an acidic amino acid E at the terminus.

In some embodiments, the hydrophilic domain comprises two β-turn regions, wherein one β-turn region comprises an acidic amino acid E at the terminus and the other β-turn region comprises an amino acid V or K at the terminus.

In some embodiments, the hydrophilic domain comprises two β-turn regions, the β-turn regions comprising an acidic amino acid D at the terminus.

In some embodiments, the hydrophilic domain comprises two β-turn regions, wherein one β-turn region comprises an acidic amino acid D at the terminus and the other β-turn region comprises an amino acid V at the terminus.

In some embodiments, the hydrophilic domain comprises at least one β-turn motif in which $X_2$ is a hydroxyproline O, or the hydrophilic domain comprises at least one β-turn motif in which $X_2$ is a proline P.

In some embodiments, the hydrophilic domain comprises at least one β-turn motif in which $X_2$ is O.

In some embodiments, the hydrophilic domain comprises at least one β-turn motif in which $X_1$ is G.

In some embodiments, the hydrophilic domain comprises a β-turn motif in which $X_2$ is O, and a β-turn motif in which $X_2$ is P.

In some embodiments, the hydrophilic domain comprises at least one β-turn motif in which $X_2$ is P.

In some embodiments, the hydrophilic domain comprises two β-turn motifs in which $X_2$ is P.

In some embodiments, one or more of the $X_1$, $X_3$ and $X_4$ are glycine (G), and/or one or two of the $X_3$ and $X_4$ are alanine (A).

In some embodiments, the β-turn motif comprises an amino acid sequence selected from the followings: GPGG (SEQ ID NO.: 33), GPGA (SEQ ID NO.: 34), GPAG (SEQ ID NO.: 35), GPG, GPAA (SEQ ID NO.: 36), GPGGG (SEQ ID NO.: 37), GOGG (SEQ ID NO.: 38), GOGA (SEQ ID NO.: 39), GOAG (SEQ ID NO.: 40), GOGGA (SEQ ID NO.: 41), GOAA (SEQ ID NO.: 42), GOG, or GOGV (SEQ ID NO.: 43).

Preferably, the β-turn motif has an amino acid sequence selected from the followings: GPAGE (SEQ ID NO.: 44), GPGGE (SEQ ID NO.: 45), GOGAE (SEQ ID NO.: 46), GOGGAE (SEQ ID NO.: 47), GOGE (SEQ ID NO.: 48), GOGGE (SEQ ID NO.: 49), GPGAD (SEQ ID NO.: 50), GOGGD (SEQ ID NO.: 51), GPGGV (SEQ ID NO.: 52), GOGGV (SEQ ID NO.: 53), GPGGK (SEQ ID NO.: 54), GOGGK (SEQ ID NO.: 55), GPGAE (SEQ ID NO.: 56), GOGAD (SEQ ID NO.: 57), GPAAD (SEQ ID NO.: 58), GOAAE (SEQ ID NO.: 59), GPGGD (SEQ ID NO.: 60), GPGGGV (SEQ ID NO.: 61), GPGV (SEQ ID NO.: 62), GOGGI (SEQ ID NO.: 63) or GOGVI (SEQ ID NO.: 64).

In some embodiments, the $X_1$ and $X_4$ form a hydrogen bond.

In some embodiments, the hydrophilic domain comprises 2, 3, 4, 5, 6, 7 or 8 β-turn motifs, preferably the hydrophilic domain comprises 2 or 3 β-turn motifs.

In some embodiments, the β-turn motif has an amino acid sequence selected from the followings: GOGG (SEQ ID NO.: 38), GPGG (SEQ ID NO.: 33), GOGA (SEQ ID NO.: 39), GOAG(SEQ ID NO.: 40), GPGA (SEQ ID NO.: 34), or GPAG (SEQ ID NO.: 35).

In the hydrophilic domain, at least one β-turn motif comprises an alanine, thereby enhancing the mechanical properties of the self-assembling peptide.

In some embodiments, a C-terminus of the hydrophilic domain can be modified with a reagent or group selected from the group consisting of: carboxylic acid, thiol, ketoate, nitrite, phosphonate, thiophosphite, carbonate, sulfate, nitrate, vinyl sulfone, amide, alcohol, aldehyde, amine, imine, maleimide, thiol, vinyl sulfone, azide, alkyne, alkene, ester, thioester, aryl, and/or silane.

An amino acid sequence of the hydrophilic domain is more hydrophilic compared to an amino acid sequence of the hydrophobic domain.

In some embodiments, the hydrophobic domain comprises 3-10 hydrophobic amino acids. Preferably, the hydrophobic domain comprises 3-7 hydrophobic amino acids, preferably, the hydrophobic domain comprises 3-5 hydrophobic amino acids. More preferably, the hydrophobic domain comprises 5 hydrophobic amino acids.

Preferably, the hydrophobic amino acid is selected from one or more of isoleucine (I), valine (V), leucine (L), phenylalanine (F) and alanine (A).

In some embodiments, the hydrophobic amino acid is selected from one or more of I, V, L, and F.

In some embodiments, a N-terminus of the hydrophobic domain is modified with a reagent or group selected from the group consisting of: acetyl, alcohol, aldehyde, amine, imine, maleimide, thiol, azide, vinyl sulfone, alkyne, alkene, ester, thioester, aryl and/or silane.

In some embodiments, the hydrophobic domain comprises:
LLLL (SEQ ID NO.: 65), FIIII (SEQ ID NO.: 66), IIIII (SEQ ID NO.: 67), IIII (SEQ ID NO.: 68), ILILI (SEQ ID NO.: 69), FLFLF (SEQ ID NO.: 70), IVIVI (SEQ ID NO.: 71), VLFIIV (SEQ ID NO.: 72), VLIII (SEQ ID NO.: 73), IVALF (SEQ ID NO.: 74), LFIVL (SEQ ID NO.: 75), FIAIV (SEQ ID NO.: 76), FIIIV (SEQ ID NO.: 77), Ac-VLFIIV (SEQ ID NO.: 78), Ac-IVIVI (SEQ ID NO.: 79), Ac_IIIII (SEQ ID NO.: 80), IIIIII (SEQ ID NO.: 81), FLIVI (SEQ ID NO.: 82), FLIIA (SEQ ID NO.: 83), FIFIF (SEQ ID NO.: 84), IFIFI (SEQ ID NO.: 85), IAILI (SEQ ID NO.: 86) or LLLLL (SEQ ID NO.: 87).

An amino acid sequence of the hydrophobic domain is more hydrophobic compared to an amino acid sequence of the hydrophilic domain.

In some embodiments, the self-assembling peptide further comprises a linker domain that provides a spacer region between the hydrophobic domain and the hydrophilic domain.

In some embodiments, the linker domain comprises 2-8 amino acid residues, preferably 4-5 amino acid residues.

In some embodiments, the linker domain comprises an amino acid with a small side chain, an amino acid with a hydroxyl in the side chain, and/or a hydrophobic amino acid away from the hydrophobic domain.

In some embodiments, the amino acid with a small side chain is selected from the group consisting of glycine (G), alanine (A), and serine (S).

In some embodiments, the amino acid with a hydroxyl in the side chain is selected from the group consisting of serine (S), threonine (T) and hydroxyproline (O).

In some embodiments, the hydrophobic amino acid away from the hydrophobic domain is selected from the group consisting of I, V, L, F, and A, and the hydrophobic amino acids of I, V, F, L, and A can be interchangeable.

In some embodiments, the linker domain has an amino acid sequence selected from the followings: GPOGI (SEQ ID NO.: 88), GPOGV (SEQ ID NO.: 89), GPOGL (SEQ ID NO.: 90), GSII (SEQ ID NO.: 91), GSGII (SEQ ID NO.: 92), GSVI (SEQ ID NO.: 93), GOII (SEQ ID NO.: 94), OGII (SEQ ID NO.: 95) or GTVI (SEQ ID NO.: 96), wherein S, T, and O are interchangeable with each other.

More preferably, the linker domain has an amino acid sequence selected from the followings: GSVL (SEQ ID NO.: 97), GSII (SEQ ID NO.: 91), GTII (SEQ ID NO.: 98), GTVI (SEQ ID NO.: 96), GOVI (SEQ ID NO.: 99), GSVI (SEQ ID NO.: 93), GSGII (SEQ ID NO.: 92), GSGVI (SEQ ID NO.: 100), GOII (SEQ ID NO.: 94), OGII (SEQ ID NO.: 95), GOGVI (SEQ ID NO.: 101) or GOGII (SEQ ID NO.: 102).

In some embodiments, one or more G are also comprised between the hydrophobic domain and the linker domain to enhance pliability and flexibility of the self-assembling peptide.

In some embodiments, the self-assembling peptide has a length of 15-50 amino acids, preferably 15-25 amino acids.

In some embodiments, the self-assembling peptide comprises 2, 3, 4, 5, 6, 7 or 8 β-turns, preferably, the self-assembling peptide comprises 2 or 3 β-turns.

In some embodiments, the self-assembling peptide has an amino acid sequence selected from SEQ ID NOs: 1-7 and SEQ ID NOs: 9-32:

IIIIIGSIIGPGGDGPGGV (SEQ ID NO.: 1);
IIIIIGSIIGPGGEGPGGV (SEQ ID NO.: 2);
IIIIIGSIIGOGGGEGPGGV (SEQ ID NO.: 3);
IIIIGSIIGOGGGEGPGGV (SEQ ID NO.: 4);
IIIIIGSIIGOGGGEGPGGGV (SEQ ID NO.: 5);
IIIIIGSIIGOGGGEGPGV (SEQ ID NO.: 6);
IIIIIGSIIGOGAEGPGGV (SEQ ID NO.: 7);
IIIIIGSIIGOGGVGPGGV (SEQ ID NO.: 9);
IIIIIIGSIIGOGAEGPGGVGPGGV (SEQ ID NO.: 10);
FLIVIGSIIGOGGEGPGGV (SEQ ID NO.: 11);
FLIIAGSIIGPGGDGOGGV (SEQ ID NO.: 12);
IIIIIGOGIIGPGGEGPGGE (SEQ ID NO.: 13);
FIFIFGTVIGPGGEGOGGV (SEQ ID NO.: 14);
IFIFIGTVIGPGGEGOGGK (SEQ ID NO.: 15);
IAILIGTVIGPGGEGOGGE (SEQ ID NO.: 16);
IVIVIGSIIGPGGDGPGGV (SEQ ID NO.: 17);
IVIVIGSIIGOGGDGPGGV (SEQ ID NO.: 18);
IVIVIGSIIGPGGEGOGGV (SEQ ID NO.: 19);
FLIVIGOGIIGOGGGEGPGGE (SEQ ID NO.: 20);
IVIVIGOGIIGOGGDGOGGV (SEQ ID NO.: 21);
IVIVIGSGIIGPGGEGPGGV (SEQ ID NO.: 22);
FIIIVGSIIGPGGEGPGGV (SEQ ID NO.: 23);

FIIIVGSIIGPGGEGPGGE (SEQ ID NO.: 24);
IIIIIGOGIIGOGGEGPGGV (SEQ ID NO.: 25);
Ac-IIIIIGSIIGPGGEGOGGV (SEQ ID NO.: 26);
FLIVIGSIIGOGAEGPGGV (SEQ ID NO.: 27);
FLIVIGSIIGOGAEGOGGV (SEQ ID NO.: 28);
LLLLLGSVLGPAGEGPAGE (SEQ ID NO.: 29):
LLLLLGPOGLGPAGEGPAGE (SEQ ID NO.: 30);
LLLLLGPOGVGPAGEGPAGE (SEQ ID NO.: 31); or
LLLLLGPOGIGPAGEGPAGE (SEQ ID NO.: 32).

The self-assembling peptide of the present invention having the above structure can be initiated by an initiator to form a three-dimensional network scaffold material.

The three-dimensional scaffold material has a nanostructure.

The initiator is a positively charged source substance which includes a substance with a positively charged group, or a positive ion.

In some embodiments, the positively charged source substance is a biomacromolecule, a drug, a functional molecule, or a small molecule such as a metal ion or an amino acid, or a mixture comprising one or more the above substances, wherein the number of hydrogen bond acceptors is fewer than the number of hydrogen bond donors under a neutral condition.

In some embodiments, the biomacromolecule includes, but is not limited to, an organic acid, a protein, a polysaccharide and a derivative thereof. The organic acid includes, but is not limited to, lactic acid, tannic acid, citric acid, and the like.

In some embodiments, the protein is selected from a protein that can provide a hydrogen ion under a neutral physiological condition.

Preferably, the protein is independently selected from a protein with an isoelectric point (PI) value below 7.0 (preferably 3.4-6.05).

In some embodiments, the protein includes, but is not limited to, fibrinogen, globulin, hemoglobin, transferrin, laminin, fibronectin, vitronectin, and the like.

In some embodiments, the polysaccharide and derivative thereof include, but are not limited to, chitin, chitosan, and the like.

In some embodiments, the drug includes, but is not limited to, an antibiotic, a dopamine, and the like; preferably, the antibiotic includes, but is not limited to, kanamycin, gentamicin, and the like.

In some embodiments, the functional molecule includes, but is not limited to, an antioxidant, a cell proliferation promoting component, and the like.

In some embodiments, the antioxidant includes, but is not limited to, a vitamin such as nicotinamide and the like.

In some embodiments, the cell proliferation promoting component includes, but is not limited to, spermine, spermidine, and the like.

In some embodiments, the metal ion includes, but is not limited to, potassium ion, calcium ion, magnesium ion, and the like.

In some embodiments, the amino acid includes, but is not limited to, an amino acid or a polymer thereof, such as: lysine, arginine, polylysine, polyarginine, and the like.

In some embodiments, the positively charged source substance is urea or nicotinamide mononucleotide.

In some embodiments, the positively charged source substance is serum, plasma, cell medium, tissue fluid of plant or animal, animal tissue, or a mixture comprising any of the above positively charged source substances.

The cell medium includes, but is not limited to: cell complete medium, animal component-free cell medium, animal protein-free cell medium, or chemically-defined cell medium.

The present invention unexpectedly found that the self-assembling peptide having the structure herein can be initiated and self-assembled into a nano-network structure under a physiological condition, cell storage and culture environment, and in the presence of common endogenous substances in the human body, and that the self-assembling peptide solution system can exert support and repair functions even in a solution state.

Accordingly, in a second aspect, the present invention provides a method for initiating the self-assembling peptide of the first aspect to form a three-dimensional network scaffold structure, wherein the method includes a step of initiation under a positively charged source initiating condition.

In some embodiments, the method comprises a step of initiation at a pH of less than 6.

The self-assembling peptide solution may spontaneously initiate to form a hydrogel at a pH of less than 6.

In some embodiments, the method comprises a step of initiation by a positively charged source substance at a pH of 6-10.

In some embodiments, the method comprises a step of initiation by a positively charged source substance at a pH of 6-8.0, preferably a pH of 6.5-7.5, preferably a pH of 7.0-7.5, more preferably a pH of 7.2-7.4.

The self-assembling peptide solution can be dissolved in a neutral or alkaline solvent, with the solution adjusted after dissolution. The solvent includes an aqueous solution of one or more solutions that can provide an alkaline environment, such as sodium bicarbonate, sodium hydroxide, potassium hydroxide, or ammonia.

Any solvent that can provide a neutral or alkaline environment can be used to dissolve the self-assembling peptide of the present invention, preferably a physiologically acceptable solution. The pH of the mixture of the protein substance and the self-assembling peptide is adjusted to a pH of 6-10, preferably to a pH of 6.0-8.0, more preferably 6.5-7.5, and most preferably 7.0-7.5.

A convenient and safe method is provided for preparing a controllable and broadly responsive self-assembling peptide hydrogel under a physiological condition. The method is suitable for laboratory, hospital, and even field scenarios. It is safe and highly maneuverable, simple and fast, no need to adjust the pH, temperature, light, salt or ionic composition of the system, and *in situ* formation of the self-assembling peptide hydrogel of the present invention within half an hour, demonstrating highly maneuverable. The gelation is initiated by endogenous substances, such as tissue fluid, cell complete medium, serum-free cell medium, and other common substances in the biomedical field and some artificially synthesized drugs, which reduces the requirements of the self-assembling peptide gelation process for the application scenarios, such as cell culture and storage, and tissue filling and repair, thereby better ensuring the biocompatibility of the self-assembling peptide hydrogel of the present invention.

In addition, due to the wide variety of initiator substances available for the present invention, it can be used in multiple application scenarios, such as a broadly responsive peptide hydrogel material used in 3D cell culture and storage, tissue engineering, regenerative medicine, and drug delivery, with a wide application prospect. For example, using the self-assembling peptide hydrogel of the present invention to culture different types of cells to achieve *in vitro* three-dimensional culture and establishing a cell model; injecting the self-assembling peptide hydrogel of the present invention loaded with cells/organoids/organs into the body of an animal to be used for *in vivo* 3D culture and tissue repair researches; or directly injecting the self-assembling peptide or peptoid solution of the present invention into the body of the animal/-human to form a peptide hydrogel with the tissue fluids to be used for wound dressing, hemostatic material, etc.; or mixing a drug/functional molecule with the self-assembling peptide or peptoid solution of the present invention to form a peptide hydrogel, which is then injected or applied topically to a wound or lesion *in vitro* or *in vivo* as a carrier for a slow-release drug or a functional factor; or mixing the self-assembling peptide hydrogel of the present invention with a cell suspension for cell preservation. Therefore, the self-assembling peptide hydrogel of the present invention has a wide range of applications and are safe, without introducing additional risk substances to the application scenario.

The present invention is also advantageous in that the self-assembling peptide of the present invention can be formulated into a polypeptide solution under a neutral condition. and such a polypeptide solution exhibits low viscosity due to maintaining a relatively weak self-assembled morphology prior to an addition of an initiator, thereby maintaining the ease of handling of the self-assembling peptide solution prior to use. The self-assembling peptide of the present invention comprises a relatively hydrophobic sequence segment and a relatively hydrophilic sequence segment. The relatively hydrophilic sequence segment comprises at least two consecutive sequence segments capable of forming a β-turn structure, wherein at least one of the sequence terminuses forming the β-turn structure is an acidic amino acid. The self-assembly mechanism is in that a self-assembly driving force provided by the mutual interactions between hydrophobic internation and β-turn structures to promote gelation. And the acidic amino acid on the relatively hydrophilic sequence segment realizes the responsiveness of the polypeptide to positively charged substance. The reason is that the hydrophilic domain of the polypeptide comprises at least one acidic amino acid, which is negatively charged under a neutral condition, and there is an electrostatic repulsion between the polypeptide molecules, which prevents the formation of a tight molecular stack. Although the hydrophobic interaction of the hydrophobic domains provides a driving force for the aggregation of molecules, the electrostatic repulsion between molecules hinders the stable and orderly arrangement. Moreover, the acidic amino acid of the hydrophilic domain is a hydrophilic amino acid, which tends to be exposed in the solution, and the structure of the β-turn reduces interference from other side chains near the acidic amino acid. Thus, the structure of the β-turn facilitats the function of the acidic amino acid. The β-turn structure makes the side chain groups of the acidic amino acids on the polypeptide more active, intensifies intermolecular repulsion, affects the arrangement of molecular, and further increases the difficulty for the self-assembling peptide to form a three-dimensional network scaffold structure. Furthermore, the self-assembling peptide molecules are given acceleration due to electrostatic interaction as they approach each other during movement, resulting in an increase in the kinetic energy of the whole system. As a result, the distribution of self-assembling peptide molecules in a polypeptide-only solution under a neutral condition is relatively disordered and fails to form a three-dimensional network scaffold material, thus leaving the whole system in a solution state.

Once a positively charged source initiator is added to such a self-assembling peptide solution, the carbonyl group on the self-assembling peptide forms an electrostatic interaction with the positively charged ion/group when present. The negative charge on the self-assembling peptide molecule is "shielded" or "neutralized", and the electrostatic repulsion between molecules is reduced. The β-turn structure in the polypeptide sequence enhances the interlocking interactions between peptides, while threonine, serine and hydroxyproline, which comprise a hydroxyl, further enhance the interactions between the polypeptides through the formation of hydrogen bond. Consequently, under the drive of hydrophobic interactions and hydrogen bond, molecules aggregate orderly and distribute regularly, forming three-dimensional network scaffold materials. The trigger mode of the polypeptide of the present invention is particularly advantageous for use in the preparation of therapeutic hydrogel for injection, which is highly convenient to mix functional components and is very easy to manipulate when injecting. When injected into tissue, the peptide rapidly self-assembles to form a gel capable of exerting a function. When conducting 3D cell culture, the polypeptide solution in a solution state is also very convenient for inoculation of cells, and when mixing with the cells, a self-assembly process can be initiated, thereby achieving a support function for the cells.

The three-dimensional network scaffold material is of a nanostructure.

The initiator is a positively charged source substance which includes a substance with a positively charged group, or a positive ion.

**In** some embodiments, the positively charged source substance is a biomolecule, a drug, a functional molecule, or a small molecule such as a metal ion or an amino acid, or a mixture comprising one or more of the above substances, wherein the number of hydrogen bond acceptors is fewer than the number of hydrogen bond donors.

In some embodiments, the biomolecule includes, but is not limited to, an organic acid, a protein, a polysaccharide and a drivative thereof. The organic acid includes, but is not limited to, lactic acid, tannic acid, citric acid, and the like.

In some embodiments, the protein is selected from a protein that can provide a hydrogen ion under a neutral physiological condition.

Preferably, the protein is independently selected from a protein with an isoelectric point (PI) value below 7.0 (preferably 3.4-6.05).

In some embodiments, the protein includes, but is not limited to, fibrinogen, globulin, hemoglobin, transferrin, laminin, fibronectin, vitronectin, and the like.

In some embodiments, the polysaccharide and derivative thereof include, but are not limited to, chitin, chitosan, and the like.

In some embodiments, the drug includes, but is not limited to, an antibiotic, a dopamine, and the like; preferably, the antibiotic includes, but is not limited to, kanamycin, gentamicin, and the like.

In some embodiments, the functional molecule includes, but is not limited to, an antioxidant, a cell proliferation promoting component, and the like.

In some embodiments, the antioxidant includes, but is not limited to, a vitamin such as nicotinamide and the like.

In some embodiments, the cell proliferation promoting component includes, but is not limited to, spermine, spermidine, and the like.

In some embodiments, the metal ion includes, but is not limited to, potassium ion, calcium ion, magnesium ion, and the like.

In some embodiments, the amino acid includes, but is not limited to, an amino acid or a polymer thereof, such as: lysine, arginine, polylysine, polyarginine, and the like.

In some embodiments, the positively charged source substance is urea or nicotinamide mononucleotide.

In some embodiments, the positively charged source substance is serum, plasma, cell medium, tissue fluid of plant or animal, or a mixture comprising any of the above positively charged source substances.

The cell medium includes, but is not limited to: cell complete medium, animal component-free cell medium, animal protein-free cell medium, or chemically-defined cell medium.

It should be noted that if the hydrophobic domain is too hydrophobic, it tends to easily form associations, while if the hydrophobic domain is not hydrophobic enough, self-assembly cannot be achieved. The hydrophilic domain interacts with the initiator to initiate the self-assembly of the self-assembling peptide aqueous solution into a peptide hydrogel. Therefore, the selection of hydrophobic amino acid in the hydrophobic domain, the selection of hydrophilic amino acid in the hydrophilic domain, and the balance and precision coordination between the hydrophobic amino acid and hydrophilic amino acid are particularly important for the self-assembling peptides of the present invention not to form a hydrogel under a neutral condition but to do so under the initiating condition.

The present invention unexpectedly found that the self-assembling peptide having said structures of the present invention can be initiated and self-assemble to form a nano-network structure under a physiological condition, cell storage and culture environment, and in the presence of common endogenous substances in the human body. In particular, the use of endogenous physiological substances, such as serum, plasma, cell medium, tissue fluid of plant and animal, and animal tissue as an initiator, significantly reduces safety risk in clinical applications, which is particularly important for drug delivery, tissue repair in the human body, and regenerative medicine.

In some embodiments, the three-dimensional network scaffold material is in a form of a hydrogel or in a dried form of a hydrogel, such as in a form of a lyophilized powder of a hydrogel.

In a third aspect, the present invention provides a three-dimensional network scaffold material in a form of a hydrogel, the three-dimensional network scaffold material comprising the self-assembling peptide of the first aspect.

In some embodiments, the three-dimensional network scaffold material is obtained using the method of the second aspect.

In some embodiments, the three-dimensional network scaffold material is in a form of a hydrogel or in a dried form of a hydrogel, such as in a form of a lyophilized powder of a hydrogel.

In some embodiments, the three-dimensional network scaffold material is in a form of an injectable hydrogel.

In some embodiments, the three-dimensional network scaffold material is of a nanostructure.

The three-dimensional network scaffold material has more β-sheet structures relative to the self-assembling peptide.

In a fourth aspect, the present invention provides a composition comprising the self-assembling peptide of the first aspect and an initiator.

The composition can be in a form of a mixture together, or in a form of a combination, and the latter is such that the self-assembling peptide and the initiator are placed in separate containers.

The initiator is a positively charged source substance which includes a substance with a positively charged group, or a positive ion.

In some embodiments, the positively charged source substance is a biomacromolecule, a drug, a functional molecule, or a small molecule such as a metal ion or an amino acid, or a mixture comprising one or more of the above endogenous or exogenous sources, wherein the number of hydrogen bond acceptors is fewer than the number of hydrogen bond donors.

In some embodiments, the biomacromolecule includes, but is not limited to, an organic acid, a protein, a polysaccharide and a derivative thereof. The organic acid includes, but is not limited to, lactic acid, tannic acid, citric acid, and the like.

In some embodiments, the biomacromolecule includes, but is not limited to, an organic acid, a protein, a polysaccharide and a derivative thereof. The organic acid includes, but is not limited to, lactic acid, tannic acid, citric acid, and the like.

In some embodiments, the protein is selected from a protein that can provide a hydrogen ion under a neutral physiological condition.

Preferably, the protein is independently selected from a protein with an isoelectric point (PI) value below 7.0 (preferably 3.4-6.05).

In some embodiments, the protein includes, but is not limited to, fibrinogen, globulin, hemoglobin, transferrin, laminin, fibronectin, vitronectin, and the like.

In some embodiments, the polysaccharide and derivative thereof include, but are not limited to, chitin, chitosan, and the like.

In some embodiments, the drug includes, but is not limited to, an antibiotic, a dopamine, and the like; preferably, the antibiotic includes, but is not limited to, kanamycin, gentamicin, and the like.

In some embodiments, the functional molecule includes, but is not limited to, an antioxidant, a cell proliferation promoting component, and the like.

In some embodiments, the antioxidant includes, but is not limited to, a vitamin such as nicotinamide and the like.

In some embodiments, the cell proliferation promoting component includes, but is not limited to, spermine, spermidine, and the like.

In some embodiments, the metal ion includes, but is not limited to, potassium ion, calcium ion, magnesium ion, and the like.

In some embodiments, the amino acid includes, but is not limited to, an amino acid or a polymer thereof, such as: lysine, arginine, polylysine, polyarginine, and the like.

In some embodiments, the positively charged source substance is urea or nicotinamide mononucleotide.

In some embodiments, the positively charged source substance is serum, plasma, cell medium, tissue fluid of plant or animal, or a mixture comprising any of the above positively charged source substances.

The cell medium includes, but is not limited to: cell complete medium, animal component-free cell medium, animal protein-free cell medium, or chemically-defined cell medium.

The present invention unexpectedly found that the three-dimensional network scaffold structure formed under a condition of multiple initiators is more stable, and thus such self-assembling peptide is particularly suitable for *in vivo* applications and has unparalleled advantages over other self-assembling peptides in the prior art. The three-dimensional network scaffold material is of a nanostructure.

In a fifth aspect, the present invention provides a use of the self-assembling peptide of the first aspect, the method of the second aspect, the 3D network scaffold material of the third aspect, and the kit of the fourth aspect for use in one or more of applications selected from the group consisting of: regenerative medicine and tissue regeneration; 2D and 3D cell culture and storage; dispersion and encapsulated filling of microspheres; drug delivery; wound healing; implantable material; gene therapy; stem cell therapy; and medical cosmetology.

**DETAILED DESCRIPTION OF THE EMBODIMENTS**

The present invention obtains a self-assembling peptide by ingenious control of a secondary structure of the polypeptide, unique selection of hydrophobic amino acid in the hydrophobic domain, unique selection of hydrophilic amino acid in the hydrophilic domain, and balance and precise coordination between hydrophobic amino acid and hydrophilic amino acid.

The self-assembling peptide can respond to common positively charged ions/groups source substances found within the human body or in cell medium under a neutral condition of human physiological condition, forming a three-dimensional nanofibrillar network structure that comprises the self-assembling peptide.

The self-assembling peptide of the present invention are in a neutral liquid state throughout their use under a human physiological condition, avoiding a risk associated with pH adjustment or the introduction of other exogenous substances, such as certain metal salt ions, specific proteins, and the like.

The self-assembling peptide of the present invention exhibits a stronger support due to the presence of hydroxyproline (O) in the β-turn motif of the hydrophilic domain. Furthermore, the present invention has found that the self-assembling peptide exhibits a stronger support in the presence of hydroxyproline (O) than in the presence of proline (P) in the β-turn motif of the hydrophilic domain.

The self-assembling peptide solution may be spontaneously initiated to form a hydrogel in a solution at a pH of less than 6.

The self-assembling peptide can form a hydrogel when a positively charged source substance is added as an initiator at a pH of 6-10.

The aqueous solution of peptide is initiated to self-assemble into a peptide hydrogel by an interaction between the acidic amino acid of the self-assembling peptide of the present invention and the initiator. Under a neutral condition, the addition of the initiator can provide positively charged substances, which can neutralize negatively charged acid radical anion on the polypeptide molecules, thus reducing repulsion force between the polypeptide molecules. The polypeptide molecules then can realize self-assembling through hydrophobic interactions and hydrogen bond, ultimately forming a three-dimensional nano-network structure.

The present invention is also advantageous in that the self-assembling peptide of the present invention can be formulated into a polypeptide solution under a neutral condition and such a polypeptide solution does not form a three-dimensional network scaffold material prior to the addition of an initiator, thereby maintaining the ease of handling of the self-assembling peptide solution prior to use. The reason is that the hydrophilic domain of the polypeptide comprises at least one acidic amino acid, which is negatively charged under a neutral condition, and there is an electrostatic repulsion between the polypeptide molecules, which prevents the formation of a tight molecular stack. Although the hydrophobic interaction of the hydrophobic domains provides a driving force for the aggregation of molecules, the electrostatic repulsion between molecules hinders the stable and orderly arrangement. Moreover, the acidic amino acid of the hydrophilic domain is a hydrophilic amino acid, which tends to be exposed in the solution, and the structure of the β-turn reduces interference from other side chains near the acidic amino acid. Thus, the structure of the β-turn facilitats the function of the acidic amino acid. The β-turn structure makes the side chain groups of the acidic amino acids on the polypeptide more active, intensifies intermolecular repulsion, affects the arrangement of molecular, and further increases the difficulty for the self-assembling peptide to form a three-dimensional network scaffold structure. Furthermore, the self-assembling peptide molecules are given acceleration due to electrostatic interaction as they approach each other during movement, resulting in an increase in the kinetic energy of the whole system. As a result, the distribution of self-assembling peptide molecules in polypeptide-only solutions under a neutral condition is relatively disordered and fails to form a three-dimensional network scaffold material.

Once a positively charged source initiator is added to such a self-assembling peptide solution, the carbonyl group on the self-assembling peptide forms an electrostatic interaction with the positively charged ion/group when present. The negative charge on the self-assembling peptide molecule is "shielded" or "neutralized", and the electrostatic repulsion between molecules is reduced. The β-turn structure in the polypeptide sequence enhances the interlocking interactions between peptides, while threonine, serine and hydroxyproline, which comprise a hydroxyl, further enhance the interactions between the polypeptides through the formation of hydrogen bond. Consequently, under the drive of hydrophobic interactions and hydrogen bond, molecules aggregate orderly and distribute regularly, forming three-dimensional network scaffold materials. The trigger mode of the polypeptide of the present invention is particularly advantageous for use in the preparation of therapeutic hydrogel for injection, which is highly convenient to mix functional components and is very easy to manipulate when injecting. When injected into tissue, the peptide rapidly self-assembles to form a gel capable of exerting a function. When conducting 3D cell culture, the polypeptide solution in a solution state is also very convenient for inoculation of cells, and when mixing with the cells, a self-assembly process can be initiated, thereby achieving a support function for the cells.

In particular, under a physiological condition, such as a neutral physiological condition, the addition of protein substance or a mixture system comprising such a protein substance can provide a positive charge, which leads to the neutralization of negatively charged acid radical anion on the self-assembling peptide molecule, thus reducing repulsion force between the self-assembling peptide molecules. The peptide molecules then can realize self-assembling through hydrophobic interactions and hydrogen bond, ultimately forming a three-dimensional nano-network structure.

Accordingly, the present invention is also advantageous in that the self-assembling peptide of the present invention can be formulated into a self-assembling peptide solution under a neutral condition and such a self-assembling peptide solution does not form a three-dimensional network scaffold material prior to an addition of a protein substance or a mixture system comprising a protein substance, thereby maintaining a stability of the self-assembling peptide solution prior to use. The reason is that the hydrophilic domain of the self-assembling peptide comprises at least one acidic amino acid, which is negatively charged under a neutral condition. There is an electrostatic repulsion between the self-assembling peptide molecules, which prevents the formation of a tight molecular stack. Although the hydrophobic interaction of the hydrophobic domains provides a driving force for the aggregation of molecules, the electrostatic repulsion between molecules hinders the stable and orderly arrangement. Moreover, the acidic amino acid of the hydrophilic domain is a hydrophilic amino acid, which tends to be exposed in the solution, and the structure of the β-turn reduces interference from other side chains near the acidic amino acid. Thus, the structure of the β-turn facilitats the function of the acidic amino acid. The β-turn structure makes the side chain groups of the acidic amino acids on the peptide more active, intensifies intermolecular repulsion, affects the arrangement of molecular, and further increases the difficulty for the self-assembling peptide to form a three-dimensional network scaffold structure. Furthermore, the self-assembling peptide molecules are given acceleration due to electrostatic interaction as they approach each other during movement, resulting in an increase in the kinetic energy of the whole system. As a result, the distribution of self-assembling peptide molecules is relatively disordered in a peptide-only solution under a neutral condition and fails to form a three-dimensional network scaffold material. This is particularly advantageous for use in the preparation of therapeutic hydrogel for injection.

Once such a self-assembling peptide solution is added with a positively charged source protein substance or a mixture system comprising a protein, the carbonyl group on the self-assembling peptide forms an electrostatic interaction with the positively charged ion/group when present. The negative charge on the self-assembling peptide molecule is "shielded", and the electrostatic repulsion between the molecules is reduced. Consequently, under the drive of hydrophobic interactions and hydrogen bond, molecules aggregate orderly and distribute regularly, forming a three-dimensional network scaffold material.

Before the addition of an initiator, the self-assembling peptide exhibits a form consisted of curved and intertwined nanofibers, with uneven fiber distribution, curved fibers, and high flexibility. In contrast, the presence of an initiator substance reduces fiber curvature, significantly increases length and diameter of fiber, and changes the arrangement of fibers, with fibers tightly and orderly arranged to form bundles of fibers that intertweave with each other. Circular dichroism (CD) analysis showed that after the addition of the initiator, the secondary structure of the nanofibers assembled by the self-assembling peptide was mainly β-turns, with an increase in β-sheets within the secondary structure of the nanofibers. This provides a favorable basis for the formation of more β-sheet structures and making the fiber structure more regular and orderly. Furthermore, the secondary structure of the fiber is more diverse, and the increase of secondary structure promotes the stability of the fiber structure.

The present invention also demonstrates that the introduction of hydroxyproline into the hydrophilic domain can significantly enhance the stability of the β-sheet formed by polypeptide self-assembly through an enhanced hydrogen bond network between polypeptides, thereby improving the stability of the self-assembled scaffold, and thus enhancing the support function for cells. Meanwhile, the β-sheet structure within the hydrophilic region enhances the hydrophobicity of the amino acid side chains without disrupting the β-turn structure of the hydrophilic region through the substitution of a single alanine for glycine, and the substitution of glutamic acid for aspartic acid. The stability of self-assembling materials is similarly increased through hydrophobic interactions, thus making the scaffold materials a stronger support for cells.

In addition, due to the overall negative charge of cell membrane of erythrocytes, in the absence of positively charged ions/groups, the self-assembling peptide cannot form an effective three-dimensional network scaffold material to provide support for cells. Under the stimulation of various types of positively charged ions/groups, the self-assembling peptide of the present invention forms a three-dimensional network scaffold material, which allows the erythrocytes to achieve a three-dimensional distribution.

The cell proliferation rate of cells grown in a two-dimensional environment exhibits a trend of gradually decreasing proliferation rate after after a period of culture, and the cell state deteriorates, followed by a sharp decline in the total number of cells. The three-dimensional network scaffold formed by self-assembly of the self-assembling peptide of the present invention allows the cells to grow at a relatively uniform and stable growth rate throughout the culture period. This indicates that the three-dimensional network scaffold material of the present invention is not only non-toxic to cells and with good biocompatibility, but also enables the cells to proliferate in a good state and stably for a long period of time under the support of the network scaffold.

The addition of the self-assembling peptide of the present invention to a cell medium containing a positively charged source natural substances can highly replicate the growth environment for cells *in vivo,* and enabling long-term three-dimensional cell culture *in vitro.*

The storage effect of the hematopoietic stem cell storage system using the three-dimensional network scaffold formed by self-assembly of the self-assembling peptide of the present invention is superior to that of conventional two-dimensional storage, significantly improving cell viability.

**Definitions:**

**Hydrogel** is a kind of hydrophilic high-molecular polymer material that can be chemically or physically cross-linked to form a three-dimensional network structure. Hydrogel material can come from a natural or synthetic source. Natural polymer, such as chitosan, alginate, hyaluronic acid (HA), collagen, and gelatin, has advantages like biodegradability and carrying integrin-binding sites, but may present immunogenicity issues. Synthetic polymers, such as polyethylene glycol (PEG), polyacrylamide (PAM), polyvinyl alcohol (PVA), and polymethylmethacrylate (PMMA) offer advantages like strong mechanical properties, customizability, and low immunogenicity, yet they lack innate biological functionality and must undergo significant post-processing in order to initiate the desired *in vivo* responses.

Hydrogel has the following properties:

1. Good biocompatibility: The high-molecular polymer comprises a large number of hydrophilic groups, can absorb dozens of times more water than its weight, has a characteristic of swelling without dissolution in water, and has a good water retention capacity.
2. Extracellular matrix-like property: Through structural design, the physical and chemical properties and mechanical performance of the hydrogel are similar to that of the extracellular matrix, which is favorable for cell growth and proliferation.
3. Biodegradability: Certain natural polymer materials are biodegradable, avoiding secondary damage caused by implant removal. These unique advantages make hydrogel stand out in biomedical materials.

In the present invention, a hydrogel is obtained by encapsulating water when a peptide having the structure of both a hydrophilic surface and a hydrophobic surface self-assembles under the said conditions of the present invention, in particular under a physiological condition.

**Self-assembling Peptide** or Peptide Self Assembly is a short chain of amino acids with alternating charged and polar domains. When dissolved in neutral solvents and physiological salt concentrations, these polypeptides spontaneously assemble into hierarchical nanostructures through hydrogen bond, ionic bond, hydrophobic interactions, or van der Waals forces. Materials derived from these components have advantages such as being non-toxic, non-immunogenic, non-thrombogenic, degradable and easily metabolized. At the same time, nanofibers have the same size scale as natural ECM fibers, can be easily designed to mimic the stiffness of various soft tissues, and can be further functionalized by cellular interaction with peptide domains or the attachment of cytokines and growth factors, and thus can be used to design biologically relevant culture environments and improve the control of proliferating cell populations.

Self-assembling peptide as a drug carrier can be used as a hydrogel for wound healing treatment, and as polypeptide nanofibers for cancer therapy for sustained release of small molecules, growth factors and monoclonal antibodies. For example, self-assembling peptide can be used in studies to promote angiogenesis within regenerative tissues and to repair skin wounds using polypeptide-based scaffolds. However, the application of self-assembling peptide in the field of injectable therapeutic hydrogels is still in its infancy.

The alternating hydrophilic and hydrophobic amino acid residues in self-assembling peptides allow the self-assembling peptides to retain a significant amount of water thereby forming hydrogels. The hydrophilic residue side chains can interact directly with water, and the water molecules form an inclusion complex to surround the hydrophobic residue side chains. The number of hydrophobic and hydrophilic residues in a self-assembling peptide needs to be skillfully designed and proportioned such that if there are too many hydrophobic residues, the self-assembling peptide will be insoluble in water and will precipitate out of the water; on the other hand, if there are too many hydrophilic residues, the self-assembling peptide will be highly watersoluble and therefore will not form a hydrogel. Moreover, it requires precise and ingenious induction of self-assembly of peptide molecules into ordered nanostructured materials such as nanofibers, nanotubes and nanovesicles.

The term "amino acid" includes those naturally occurring as well as non-naturally amino acids, such as D-form natural amino acid, $\beta$ and $\gamma$ derivatives. In accordance with standard nomenclature, amino acid residue sequence is denominated by either a three letter or a single letter code, for example: Alanine (Ala, A); Arginine (Arg, R); Asparagine (Asn, N); Aspartic acid (Asp, D); Cysteine (Cys, C); Glutamine (Gln, Q); Glutamic acid (Glu, E); Glycine (Gly, G); Histidine (His, H); Isoleucine (Ile, I); Leucine (Leu, L); Lysine (Lys, K); Methionine (Met, M); Phenylalanine (Phe, F); Proline (Pro, P); Serine (Ser, S); Threonine (Thr, T); Tryptophan (Trp, W); Tyrosine (Tyr, Y); Valine (Val, V); Selenocysteine (Sec, U); and Hydroxyproline (Hyp, O).

The term "peptide" in the present invention is a chain of amino acids. In particular, the peptide has 2 to 40 amino acids in length.

The term "self-assembly" in the present invention refers to a process of polypeptide to aggregate and form an ordered structure under a normal environmental condition.

The term "β-sheet" in the present invention refers to a more extended, periodically folded, serrated main-chain conformation of the polypeptide chain, arranged in a parallel or antiparallel arrangement to form a β-sheet (or β-strand). A parallel or anti-parallel conformation is determinated based on the arrangement of the peptide direction from N to C terminus. In parallel arrangement, all peptides are aligned in the same direction from N to C terminus. In anti-parallel conformation, peptide chains are aligned in opposite direction (i.e the first peptide is aligned from N to C terminus, while the relatived second peptide is aligned from C to N terminus). Parallel arrangement can involve peptide shifing resulting in peptide termini that are staggered relative to one another. At least half the length of the peptide is involved in interpeptide interactions. In anti-parallel arrangement, polypeptides typically align in a line to provide flush ends. This is typically indicative of end-to-end complementary peptides.

A β-turn is an irregular secondary structure in proteins that causes a change in the direction of the polypeptide chain. β-turn often occurs at a point where the peptide chain makes a 180° reversal. A β-turn consists of 3-5 amino acid residues, with the second residue being either proline (P) or hydroxyproline (O). A β-turn motif is usually stabilized by the formation of a hydrogen bond between the carbonyl oxygen atom of the $n^{th}$ amino acid residue and the amide proton of the $n+3^{rd}$ amino acid residue. β-turn, also known as β-bend, reverse bend, or β-loop, is used to connect β-chain.

The term "hydrophobicity" in the present invention refers to the property of tending to repel water or to be incapable of completely dissolving in water.

The term "hydrophilic" in the present invention refers to the property of being able to readily absorb moisture and having strongly polar groups that readily interact with water.

Hydrophilic amino acid, i.e. polar amino acid, has polar R-group that can form hydrogen bond with water molecule, thereby showing affinity for water. Hydrophilic amino acid includes: S, T, Y, C, U, N, Q, D, E, O, R, K, and H.

Hydrophobic amino acid, or non-polar amino acid, has non-polar R group with low or very low affinity for water molecule, but high affinity for liposoluble substance. They include: G, A, V, L, I, P, M, F and W.

The term "nanostructure" refers to a structure with nanometer scale. Nanostructures can be any shape of one dimensional (1D), two dimensional (2D), or three dimensional (3D) space, including nano-thin film, nanofibril, nanorod, nanowire, nanofiber network, nanosphere, nanohelix, and a mixture of several of them. The surface of nanostructure has one dimension on the nanoscale, i,e, the thickness of the surface of an objcct is between 0.1 nm and 100 nm. Nanotube has two dimensions on the nanoscale, i.e., the diameter of the nanotube is between 0.1 nm and 100 nm; and its length could be much greater. Spherical nanoparticle has three dimensions on the nanoscale, ie., the size of the nanoparicle is between 0.1 nm and 100 nm in each spatial dimension.

When a protein solution is at a certain pH, the protein dissociates into positive and negative ions with equal tendency, i.e., it becomes an amphoteric ion with a zero net charge, and the pH of the solution at this point is known as the isoelectric point (pI) of the protein.

BRIEF DESCRIPTION OF THE DRAWINGS

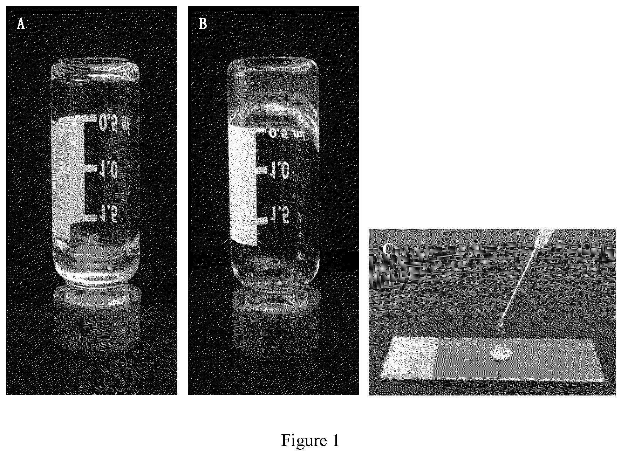

Figures 1A-1B show that when arginine was used as an initator, the self-assembling peptide was in a liquid state (A) without the initiator, and the self-assembling peptide was a hydrogel (B) in the presence of arginine; and Figure 1C shows that when the tissue liquid was used as an initator, the self-assembling peptide can form a hydrogel and maintain a gel state after being extruded by a syringe.

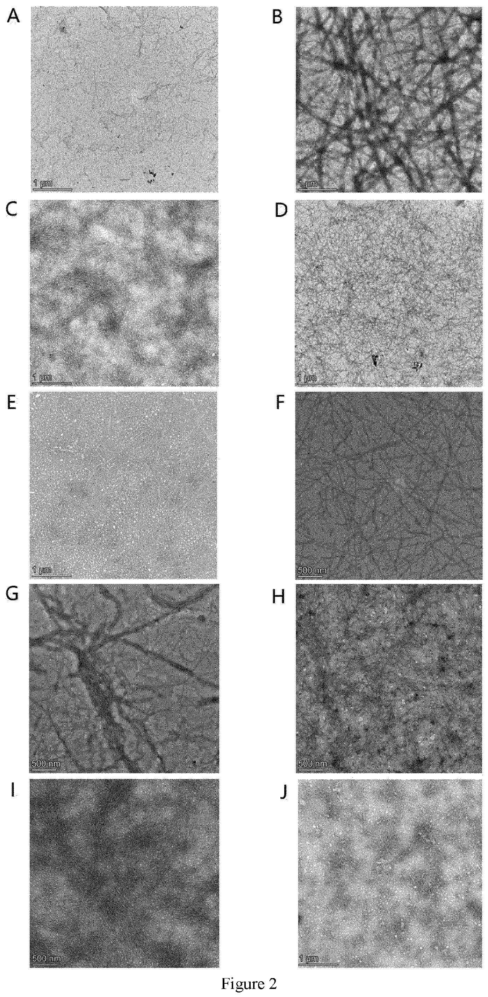

Figures 2A-2J show that different initiator substances mixed with self-assembling peptides can all yield the three-dimensional network scaffold material of the present invention.

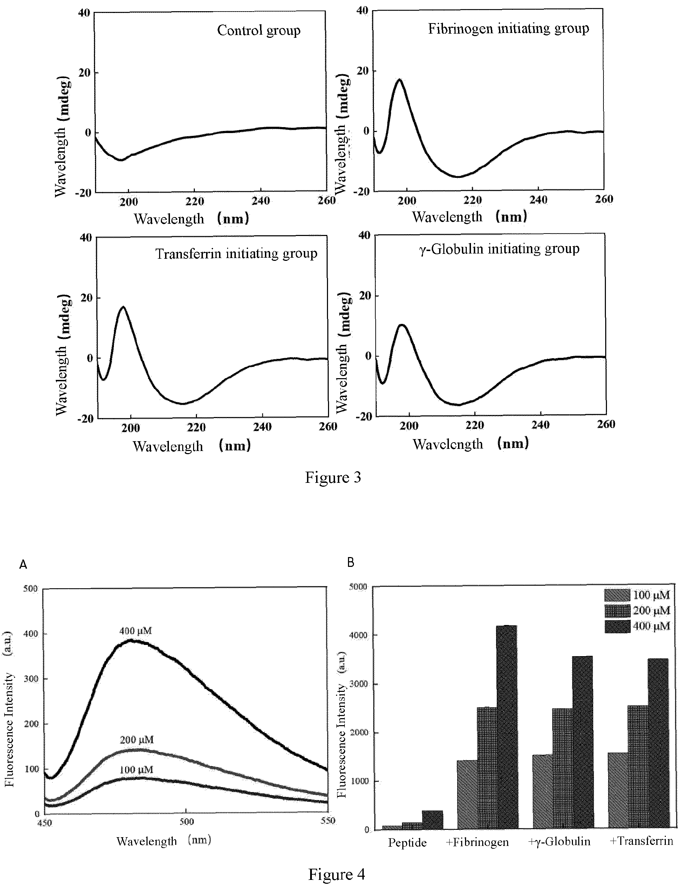

Figure 3 shows hydrogel three-dimensional network scaffold material formed with fibrinogen, transferrin, or γ-globulin as an initiator.

Figures 4A-4B show the fluorescence changes of Thioflavin T of peptide fiber hydrogels formed with fibrinogen, transferrin, or γ globulin as an initiator.

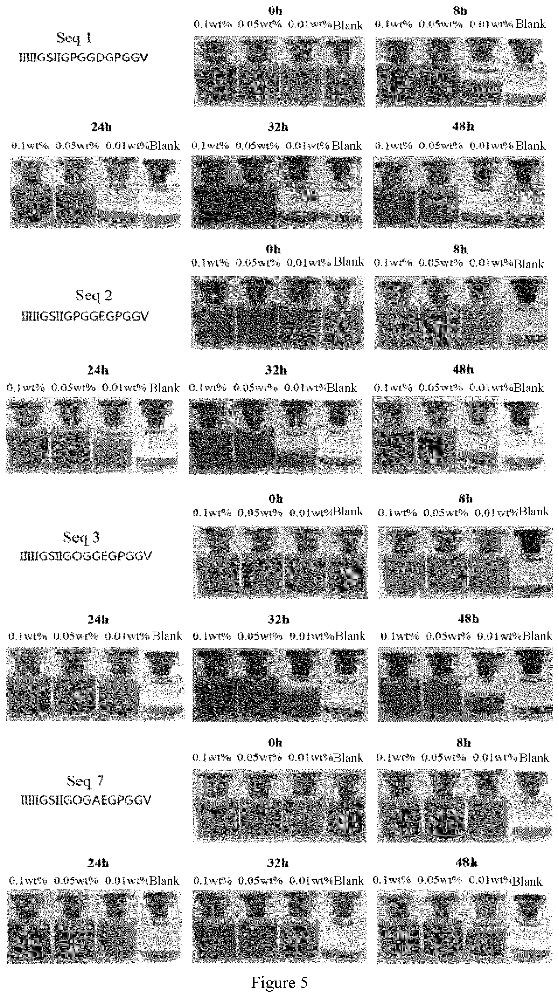

Figure 5 shows the effect of different sequence structures of the self-assembling peptides on the support for erythrocytes in forming a three-dimensional network scaffold.

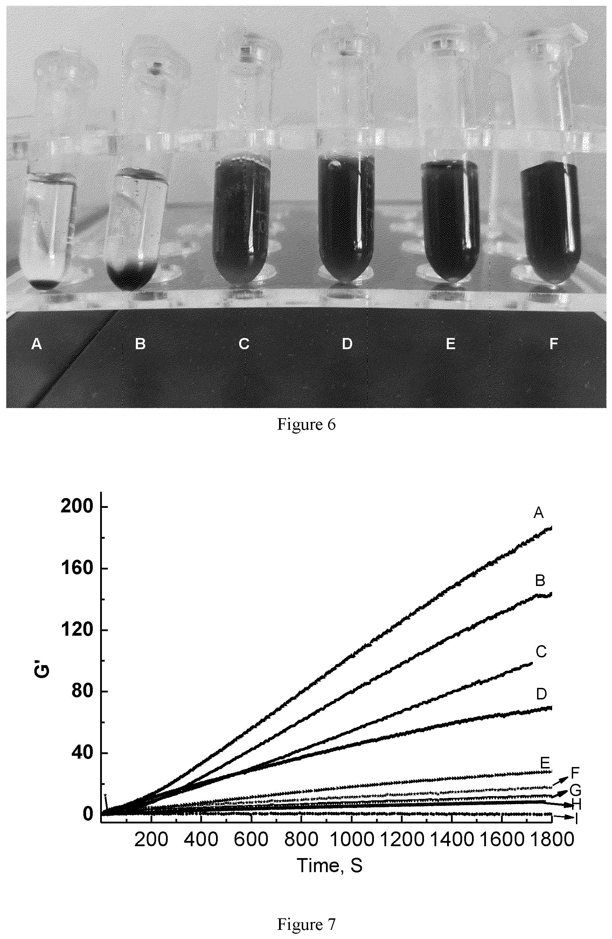

Figure 6 shows the effect of the initiating components on the support for erythrocytes by self-assembling peptide solutions.

Figure 7 shows the rheological property changes of different sequences of self-assembling peptide. These are sequentially SEQ ID NO:7 (A), SEQ ID NO:3 (B), SEQ ID NO:2 (C), SEQ ID NO:1 (D), SEQ ID NO:5 (E), SEQ ID NO:6 (F), SEQ ID NO:4 (G), SEQ ID NO:10 (H), and SEQ ID NO:8 (I).

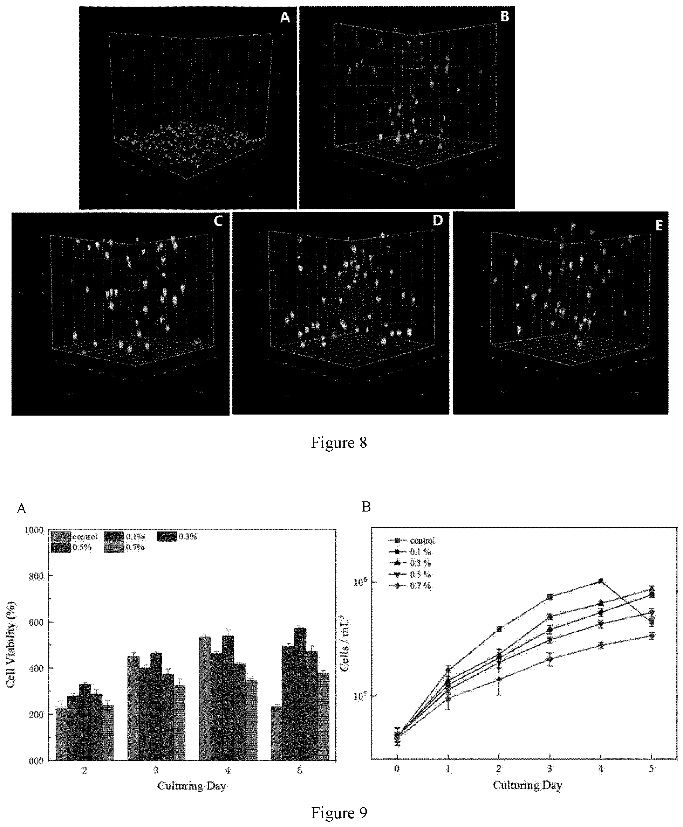

Figures 8A-8E show confocal laser scanning electron microscopy images of the effect of self-assembling peptide materials of the present invention on supporting cells after being initiated by spermine (B), high glucose complete medium (C), tissue fluid (D), and serum (E), respectively, wherein the concentration of the self-assembling peptide in all cases was 0.1 wt.%, with (A) being a buffer without any initiating component.

Figures 9A-9B show the relative viability over a period of 5 days (A) and the growth curves over a period of 5 days (B) of cells cultured using different concentrations of the self-assembling peptide of the present invention that were initiated to form a 3D network scaffold material in complete medium.

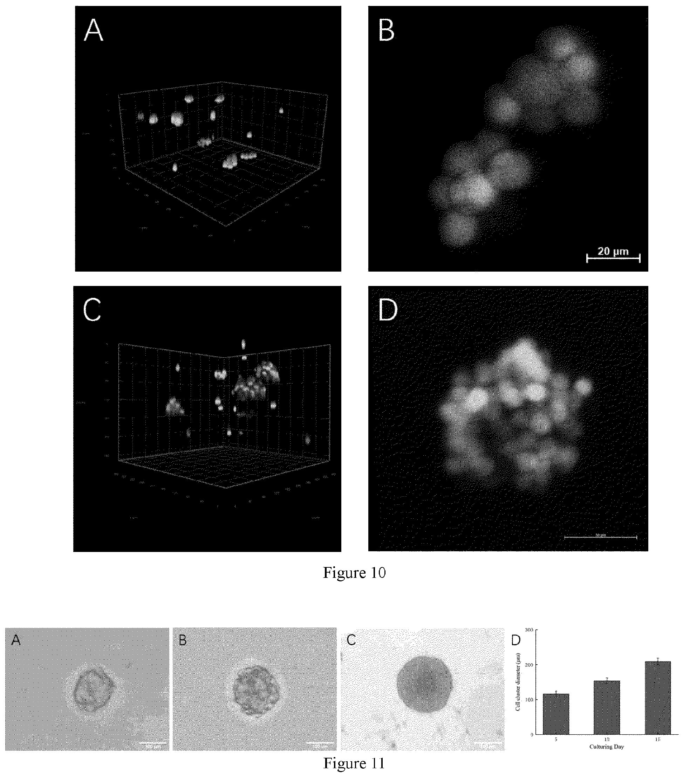

Figures 10A-10D show confocal laser scanning electron microscopy images of cells cultured for 3 days (A) and 7 days (C), in a cell culture system where the self-assembling peptide material of the present invention was added and initiated to assembly into a fibrous network scaffold, wherein (B) and (D) are representative of typical cellular clusters in the system of cultured cells at 3 and 7 days, respectively. The concentration of the self-assembling peptide material used in the respective experimental groups was 0.1 wt. %.

Figures 11A-11D show the addition of 0.1 wt.% of the self-assembling peptide material of the present invention to a cell culture system to initiate its assembly into a fiber network scaffold and culture the cells, the cell spheroid diameters were counted on the fifth, tenth, and fifteenth days (A), and the characteristic cell spheroids in the culture system were photographed (B, C, and D).

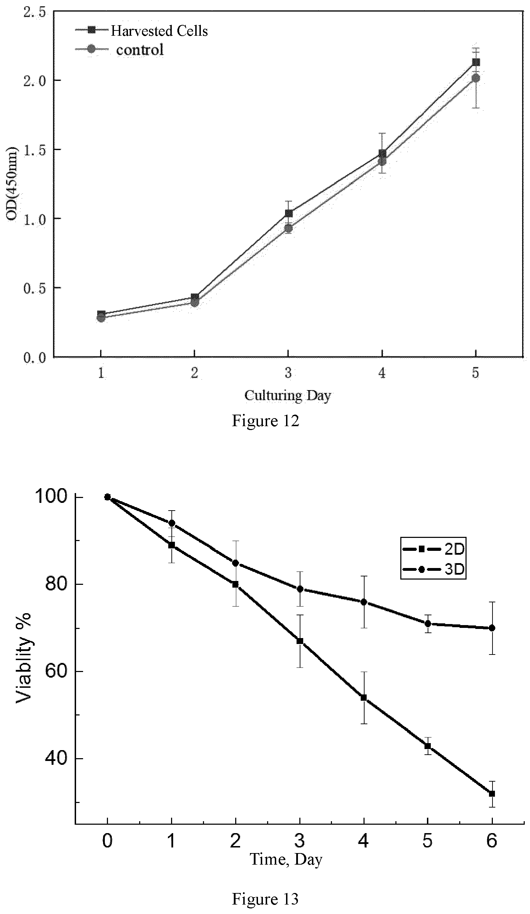

Figure 12 shows the addition of 0.1 wt.% of the self-assembling peptide material of the present invention to a cell culture system to initiate its assembly into a fiber network scaffold and culture the cells, after 15 days the cells were harvested to measure the relative viability of cells over 5 days.

Figure 13 shows the effect on cell survival during the cryopreserved storage without the self-assembling peptide scaffold solution (2D) and with the same (3D) for mouse mesenchymal stem cells.

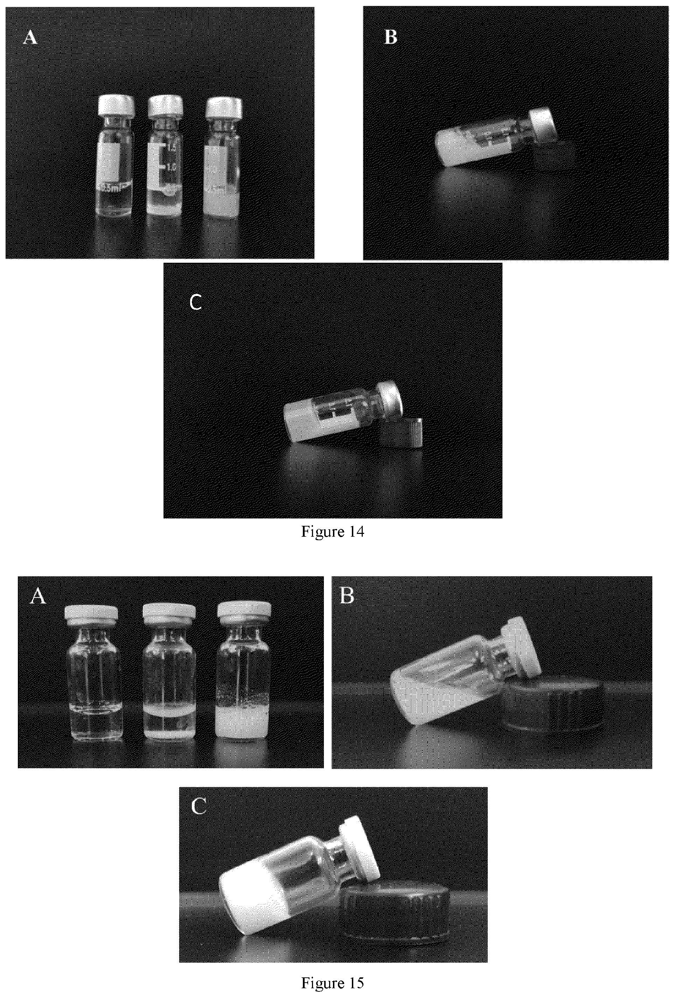

Figures 14A-14C show that self-assembling peptide solution can stably disperse L-polylactic acid microspheres, wherein A shows that the microspheres were precipitated in an aqueous solution, while they were uniformly suspended and dispersed after mixing with the self-assembling peptide solution; B shows that the protein-responsive self-assembling peptide mixture of the microspheres was in a liquid state; and C shows that upon mixing the protein-responsive self-assembling peptide mixture of the microspheres obtained in Figure 14B with a tissue fluid, the protein-responsive self-assembling peptide/L-polylactic acid microsphere solution rapidly formed a hydrogel.

Figures 15A-15C show that protein-responsive self-assembling peptide solution can stably disperse polycaprolactone: A shows that the microspheres were precipitated in an aqueous solution, while they were uniformly suspended and dispersed after mixing with the self-assembling peptide solution; B shows that the protein-responsive self-assembling peptide mixture of the microspheres was in a liquid state; and C shows that upon mixing the protein-responsive self-assembling peptide mixture of the microspheres obtained in B with a tissue fluid, the protein-responsive self-assembling peptide/L-polylactic acid microsphere solution rapidly formed a hydrogel.

## Examples

### Example 1. Synthesis of Self-assembling Peptide Compound

The self-assembling peptide or peptoid compounds of the present invention were synthesized through a solid-phase peptide synthesis method with the amino acid sequences listed as SEQ ID NOs: 1-32.

### Example 2. Preparation of Polar Peptide or Peptoid Solutions

The self-assembling peptide or peptoid of Example 1 was added to water or phosphate (PBS) buffer (pH=7.2-7.4, unless otherwise specified, the PBS buffer used herein were all for this pH). Alkaline solution was added dropwise until the self-assembling peptide was completely dissolved, and the solution was adjusted to a neutral pH of 7.2, obtaining a 5wt.% stock solution of self-assembling peptide or peptoid. This stock solution was then sterilized by autoclaving and stored at 4°C for later use. The concentration of the self-assembling peptide was adjusted by diluting the stock solution to obtain a self-assembling peptide or peptoid material with predetermined concentration. An appropriate amount of the stock solution was taken and diluted with PBS buffer to yield 0.5wt.% of self-assembling peptide or peptoid solution.

### Example 3. Preparation of Three-Dimensional Network Scaffold Material Formed by Self-assembling Peptide Stimulated by Positively Charged Ions/Groups

The self-assembling peptide or peptoid solution obtained in Example 2 was mixed homogeneously with an initiator substance solution, and the concentration ratio of the self-assembling peptide or peptoid to the initiator substance in the mixed solution was ensured within the range of (1-100):(1-100), to obtain a controllable self-assembling peptide hydrogel material with being initated by multiple initiator substances, self-assembling, 3D network scaffold, and the pH value of the resulting mixed solution was neutral (about pH 6 to about pH 8, preferably about pH 6.5-7.5, and preferably about pH 7-7.5).

Taking the self-assembling peptide shown by amino acid sequence IIIIIGSIIGPGGDGPGGV (SEQ ID NO.: 1) as an example and arginine as the initiator, the 0.5 wt.% peptide solution (see Figure 1A) was in a liquid state, and after the sample vial was inverted, the solution flowed backwards and concentrated at the mouth of the vial, and in the presence of arginine, the 0.5 wt.% of peptide concentration responded to the initiator substance arginine and formed a self-assembling peptide hydrogel (see Figure 1B). When the sample vital was inverted, the material did not fall out due to the formation of a hydrogel. Figure 1C shows that when the tissue liquid was used as the initator, the self-assembling peptide can form a hydrogel and maintain a gel state after being extruded by a syringe. Peptide hydrogel materials formed in response to other types of initiator substances had equivalent or similar effects.

However, taking the self-assembling peptide shown by amino acid sequence LLLLLGSVLGPAGEGPAGE (SEQ ID NO: 29) as an example, the self-assembling peptide with a 2% concentration rapidly forms a hydrogel when mixed with an equal volume of human tissue fluid. The hydrogel can be aspirated using a syringe and and maintains a gel state even after being extruded by the syringe. Peptide hydrogel materials formed in response to other types of initiator substances had also equivalent or similar effects.

Using this method, self-assembling peptide hydrogel materials of the present invention can be configured with any ratios of the predetermined peptide concentration of self-assembling peptide or peptoid substance to the initiator concentration. The results are summarized in Table 1 below.

Table 1. Interaction Examples Between Initiator Substances and Polar Peptides or Peptoid

| SEQ ID NO.: | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 |
|---|---|---|---|---|---|---|---|---|---|---|---|---|---|---|---|---|---|---|---|---|---|---|---|---|---|---|---|---|---|---|---|---|
| Lysine | √ | √ | √ | ~ | √ | ~ | √ | × | o | ~ | √ | √ | √ | √ | √ | √ | √ | √ | √ | √ | √ | √ | √ | √ | √ | √ | √ | √ | √ | √ | √ | √ |
| Argnine | √ | √ | √ | ~ | √ | ~ | √ | × | o | ~ | √ | √ | √ | √ | √ | √ | √ | √ | √ | √ | √ | √ | √ | √ | √ | √ | √ | √ | √ | √ | √ | √ |
| Gentamycin | √ | √ | √ | ~ | √ | ~ | √ | × | o | ~ | √ | √ | √ | √ | √ | √ | √ | √ | √ | √ | √ | √ | √ | √ | √ | √ | √ | √ | √ | √ | √ | √ |
| Kanamycin | √ | √ | √ | ~ | √ | ~ | √ | × | o | ~ | √ | √ | √ | √ | √ | √ | √ | √ | √ | √ | √ | √ | √ | √ | √ | √ | √ | √ | √ | √ | √ | √ |
| Spermine | √ | √ | √ | ~ | √ | ~ | √ | × | o | ~ | √ | √ | √ | √ | √ | √ | √ | √ | √ | √ | √ | √ | √ | √ | √ | √ | √ | √ | √ | √ | √ | √ |
| Spermidine | √ | √ | √ | ~ | √ | ~ | √ | × | o | ~ | √ | √ | √ | √ | √ | √ | √ | √ | √ | √ | √ | √ | √ | √ | √ | √ | √ | √ | √ | √ | √ | √ |
| Polylysine | √ | √ | √ | ~ | √ | ~ | √ | × | o | ~ | √ | √ | √ | √ | √ | √ | √ | √ | √ | √ | √ | √ | √ | √ | √ | √ | √ | √ | √ | √ | √ | √ |
| Chitosan | √ | √ | √ | ~ | √ | ~ | √ | × | o | ~ | √ | √ | √ | √ | √ | √ | √ | √ | √ | √ | √ | √ | √ | √ | √ | √ | √ | √ | √ | √ | √ | √ |
| Polyarginine | √ | √ | √ | ~ | √ | ~ | √ | × | o | ~ | √ | √ | √ | √ | √ | √ | √ | √ | √ | √ | √ | √ | √ | √ | √ | √ | √ | √ | √ | √ | √ | √ |
| Magnesium Ion | √ | √ | √ | ~ | √ | ~ | √ | × | o | ~ | √ | √ | √ | √ | √ | √ | √ | √ | √ | √ | √ | √ | √ | √ | √ | √ | √ | √ | √ | √ | √ | √ |
| Serum | √ | √ | √ | ~ | √ | ~ | √ | × | o | ~ | √ | √ | √ | √ | √ | √ | √ | √ | √ | √ | √ | √ | √ | √ | √ | √ | √ | √ | √ | √ | √ | √ |
| Plasma | √ | √ | √ | ~ | √ | ~ | √ | × | o | ~ | √ | √ | √ | √ | √ | √ | √ | √ | √ | √ | √ | √ | √ | √ | √ | √ | √ | √ | √ | √ | √ | √ |
| Urea | √ | √ | √ | ~ | √ | ~ | √ | × | o | ~ | √ | √ | √ | √ | √ | √ | √ | √ | √ | √ | √ | √ | √ | √ | √ | √ | √ | √ | √ | √ | √ | √ |
| Nicotinamide Mononucleotide | √ | √ | √ | ~ | √ | ~ | √ | × | o | ~ | √ | √ | √ | √ | √ | √ | √ | √ | √ | √ | √ | √ | √ | √ | √ | √ | √ | √ | √ | √ | √ | √ |
| Gelatin Microcarrier with surface positively charged modification | √ | √ | √ | ~ | √ | ~ | √ | × | o | ~ | √ | √ | √ | √ | √ | √ | √ | √ | √ | √ | √ | √ | √ | √ | √ | √ | √ | √ | √ | √ | √ | √ |
| Human Tissue Fluid | √ | √ | √ | ~ | √ | ~ | √ | × | o | ~ | √ | √ | √ | √ | √ | √ | √ | √ | √ | √ | √ | √ | √ | √ | √ | √ | √ | √ | √ | √ | √ | √ |
| Cell Complete Medium | √ | √ | √ | ~ | √ | ~ | √ | × | o | ~ | √ | √ | √ | √ | √ | √ | √ | √ | √ | √ | √ | √ | √ | √ | √ | √ | √ | √ | √ | √ | √ | √ |
| Animal Component-free Cell Medium | √ | √ | √ | ~ | √ | ~ | √ | × | o | ~ | √ | √ | √ | √ | √ | √ | √ | √ | √ | √ | √ | √ | √ | √ | √ | √ | √ | √ | √ | √ | √ | √ |
| Animal Protein-free Cell Medium | √ | √ | √ | ~ | √ | ~ | √ | × | o | ~ | √ | √ | √ | √ | √ | √ | √ | √ | √ | √ | √ | √ | √ | √ | √ | √ | √ | √ | √ | √ | √ | √ |
| Chemical Defined Cell Medium | √ | √ | √ | -- | √ | -- | √ | × | o | -- | √ | √ | √ | √ | √ | √ | √ | √ | √ | √ | √ | √ | √ | √ | √ | √ | √ | √ | √ | √ | √ | √ |
| Fibrinogen | √ | √ | √ | ~ | √ | ~ | √ | × | o | ~ | √ | √ | √ | √ | √ | √ | √ | √ | √ | √ | √ | √ | √ | √ | √ | √ | √ | √ | √ | √ | √ | √ |
| γ Globulin | √ | √ | √ | ~ | √ | ~ | √ | × | o | ~ | √ | √ | √ | √ | √ | √ | √ | √ | √ | √ | √ | √ | √ | √ | √ | √ | √ | √ | √ | √ | √ | √ |
| Transferrin | √ | √ | √ | -- | √ | -- | √ | × | o | -- | √ | √ | √ | √ | √ | √ | √ | √ | √ | √ | √ | √ | √ | √ | √ | √ | √ | √ | √ | √ | √ | √ |
| Laminin | √ | √ | √ | ~ | √ | ~ | √ | × | o | ~ | √ | √ | √ | √ | √ | √ | √ | √ | √ | √ | √ | √ | √ | √ | √ | √ | √ | √ | √ | √ | √ | √ |
| Fibronectin | √ | √ | √ | ~ | √ | ~ | √ | × | o | ~ | √ | √ | √ | √ | √ | √ | √ | √ | √ | √ | √ | √ | √ | √ | √ | √ | √ | √ | √ | √ | √ | √ |
| Vitronectin | √ | √ | √ | ~ | √ | ~ | √ | × | o | ~ | √ | √ | √ | √ | √ | √ | √ | √ | √ | √ | √ | √ | √ | √ | √ | √ | √ | √ | √ | √ | √ | √ |
| Human Collagen | × | × | × | × | × | × | × | × | o | × | × | × | × | × | × | × | × | × | × | × | × | × | × | × | × | × | × | × | × | × | × | × |
| Hemoglobin | ~ | ~ | ~ | ~ | ~ | ~ | ~ | × | o | ~ | ~ | ~ | ~ | ~ | ~ | ~ | ~ | ~ | ~ | ~ | ~ | ~ | ~ | ~ | ~ | ~ | ~ | ~ | ~ | ~ | ~ | ~ |

Note: "√" in Table 1 indicates that the self-assembling peptide mixed with an initiator in the centrifuge tube can form a peptide hydrogel within 30 minutes, and the hydrogel does not fall off when the centrifuge tube is inverted; "×" indicates that no gel can be formed, remaining in solution form; "~" indicates that a weak gel is formed, and it will fall off when inverted although it has poor fluidity; and "o" indicates that the gel can be formed by spontaneous initiation without the initiator component.

**Example 4. Structural Characterization of Scaffold Materials Formed by Self-Assembling Peptide of the Present Invention**

**Experiment 1. Transmission Electron Microscopy (TEM)**

The polar peptide or peptoid was exemplified by SEQ ID NO.: 3 (IIIIIGSIIGOGGGEGPGGV); and the initiator substances were exemplified by polylysine, spermine, gentamicin, lysine, arginine, spermidine, kanamycin, chitosan, and magnesium ions.

Experimental Methods: The stock solution of self-assembling peptide material obtained in Example 3 (SEQ ID NO.: 3) and the three-dimensional network scaffold material formed with the self-assembling peptide in response to initiator substances obtained in Example 2 were respectively diluted with ultrapure water. To obtain the nanoscale morphology of hydrogel under a physiological condition, the solutions were incubated at 37°C for 1 hour. Then, 10 μL of the solution was taken and placed on a 300-mesh carbon support film copper grid (Beijing XXBR Technology Co., Ltd), followed by vacuum drying. The samples were stained using 2 wt.% of phosphotungstic acid negative staining solution for 60 s each time and repeated three times. The stained copper grid was left to dry at room temperature. Imaging was performed using a Talos G2 200X transmission electron microscope.

Experimental Results: As shown in Figures 2A-2J, the three-dimensional network scaffold materials of the present invention can be obtained by mixing different initiator substances with the self-assembling peptides, and the initiator substances corresponding to A-J in order were: polylysine, spermine, gentamicin, lysine, arginine, spermidine, kanamycin, chitosan, and magnesium ion. The structure formed by the self-assembling peptide material (Figure 2A) consisted of curved and interwoven nanofibers with uneven fiber distribution, curved fibers, and high flexibility. The presence of initiator substances reduced the curvature of the fibers (Figures 2B-2J), significantly increasing fiber length and diameter. Moreover, the arrangement of fibers changed. The fibers are arranged tightly and orderly to form fiber bundles, and fiber bundles are interwoven with each other.

**Experiment II. Circular Dichroism (CD) Spectroscopy Analysis**

Circular dichroism spectroscopy is the most widely used method for determining the secondary structure of proteins and monitoring the conformational changes of protein molecules induced by external conditions. This method is used to detect liquids, and the results obtained closely mimic the true physiological environment of protein secondary structures, offering a rapid, simple, and relatively accurate method for studying protein conformations.

Experimental Methods: preparation of self-assembling peptide or its derivative solution for CD assay: the obtained 0.2 wt% of self-assembling peptide solution was diluted with PBS buffer to a self-assembling peptide concentration of 0.02 wt%.

Preparation of protein solutions for CD assay: laminin, fibronectin, fibrinogen, transferrin, γ globulin, and vitronectin were added to ultrapure water to a protein concentration of 0.02 wt%, respectively.

Preparation of samples of self-assembling peptides or derivatives thereof: 0.2 wt% of the self-assembling peptide solution obtained in Example 2 was diluted with PBS buffer to a self-assembling peptide concentration of 0.01 wt%, and incubated at 37°C for one hour.

Preparation of peptide hydrogel samples of the present invention: 0.02 wt% of self-assembling peptide solution was mixed with 0.02 wt% of protein solution respectively in a volume ratio of 1:1, and incubated at 37°C for one hour to obtain a mixed system with a final concentration of self-assembling peptide of 0.01 wt%. Although the concentration of self-assembling peptide in this experiment was lower than 0.1 wt%, due to the swollen nature of the self-assembling peptide/protein hydrogels, the result still validated the interaction between the self-assembling peptide and the proteins.

Separately, 400 μL of each of the prepared samples were loaded into a rectangular quartz cell with an optical path length of 1 mm. Their respective circular dichroic detection was carried out using a MOS-450/AF-CD spectrometer (Bio-Logic, Claix, France) with a resolution of 0.5 nm and a scan speed of 0.5 nm/s over the wavelength range of 190-260 nm at room tempreature. Each detection included background correction and was repeated three times. Taking the hydrogels formed with the self-assembling peptide as shown in SEQ ID NO.: 13 (IIIIIGOGIIGPGGEGPGGE) and fibrinogen, transferrin, and γ-globulin as examples, and the results were shown in Figure 3.

The results of the circular dichroic assay indicated that the response of self-assembling peptides or derivatives thereof to proteins exhibited change in their secondary structure. Figure 3 shows that the secondary structure of nanofibers assembled from self-assembling peptides or derivatives thereof when they are alone in a neutral environment (see control group) was mainly β-turn, whereas within the system in which proteins were introduced separately, the positive peaks near 200 nm in the CD spectra shifted to the right to varying degrees. This indicated that the response to proteins increased the β-sheet in the secondary structure of nanofibers assembled by self-assembling peptides or derivatives thereof, compared to when the self-assembling peptides or derivatives thereof existed alone without being initiated, which provided a favorable basis for the formation of more β-sheet structures and a more regular and ordered fiber structures. Additionally, the group of addition of protein (see fibrinogen initiating group, transferrin initiating group, γ-globulin initiating group) showed a negative peak near 192 nm. This was the manifestation of the characteristic polymeric structure formed by the participation of proline or hydroxyproline in the assembly of the self-assembling peptide in the secondary structure of the fibers. It demonstrated that the response to the three substances made the secondary structure of the fibers assembled from self-assembling peptide of the present invention more diversified, with increased secondary structure promoting stabilization of the fiber structure. This experiment illustrated that, although self-assembling peptides or derivatives thereof can themselves assemble into nanofibers with specific secondary structures, the responsiveness to proteins allowed the self-assembling peptides or derivatives thereof to assemble into nanofibers with a more complex and ordered structure, thus exhibiting macroscopically a tightly cross-linked, uniformly porous three-dimensional network structure such as those observed in Experiment I.

Experimental Conclusion: Experiment II demonstrated that the presence of the initiator substance allowed the self-assembly of the polar peptides to form a common network distribution of the gel that facilitates cell adhesion and three-dimensional culture. Combining Experiments I and II, it can be established that the interaction between the self-assembling peptide and the initiator substances changed the path of self-assembly of the self-assembling peptide, which prompted the self-assembling peptide to form a three-dimensional network scaffold material, forming a microscopic morphology that was very different from that formed without initiators.

When a self-assembling peptide or peptoid of the present invention was employed, or when a self-assembling peptide with a structure conforming to the structure of the present invention was employed, experimental results similar to those of Experiments I and II could be obtained under stimulation of the initiator substances. The specifics of each case were not given here.

### Experiment III. Thioflavin T Fluorescence Assay

Experimental Methods: Thioflavin T can bind to the β-sheet structure in the self-assembling peptide structure, thereby enhancing fluorescence intensity. In this experiment, the peptide as shown in SEQ ID NO.: 13 (IIIIIGOGIIGPGGEGPGGE) was used to verify the secondary structure changes of the self-assembling peptide during the self-assembly process by detecting the fluorescence changes of thioflavin T. Polypeptide sols at concentrations of 800μM (0.148 wt%), 400μM (0.072 wt%), and 200μM (0.036 wt%) were mixed with 0.072 wt% of initiator in a volume ratio of 1:1. For the control group, ultrapure water was used instead of the initiator. After standing for 15 minutes, it was mixed with an equal volume of 100μM thioflavin T solution. Fluorescence emission measurements were performed over the range of 450-550 nm, with five spectra collected for each sample. The excitation wavelength was 442 nm and the excitation and emission slits were 5 nm and 2.5 nm, respectively.

Experimental Results: The presence of the β-sheet was confirmed by the validation of the CD spectral data using thioflavin T (ThT), which is capable of staining peptide fibers abundant in the β-sheet structure, and the relationship of β-sheet with peptide concentration was revealed. The β-sheet was presented in the self-assembling peptide sols, its content was positively correlated with the concentration of the self-assembling peptide, indicating that the self-assembling peptide was a concentration-dependent self-assembling peptide (Figure 4A). Due to the fact that the increase in peptide monomers facilitated intermolecular contact and thus improved the degree of assembly. The β-sheet content in the gel formation groups after addition of the initiator was significantly higher than that in the sol group (Figure 4B). Both CD and ThT staining data indicated that the supramolecular polymers generated from protein initiator-activated self-assembling peptides under a neutral pH condition exhibit typical β-sheet structures.

### Example 5. Effect of Amino Acid Sequence Structure and Initiator Components on the Physical and Functional Properties of the Self-Assembling Peptide Materials of the Present Invention, and Validation of Self-Assembly Mechanism

### Experiment I. Verification of Erythrocyte Support by Different Sequences under Liquid Conditions

In this experiment, taking erythrocytes as an example, the effect of amino acid sequences on the support for cells was verified, especially the effect of hydroxyproline and alanine in the amino acid sequences on the support for cells.

Experimental Methods: PBS buffer was used to separately prepare a concentration of 0.1 wt% stock solutions of self-assembling peptides as shown in SEQ ID NO.: 1 (IIIIIGSIIGPGGDGPGGV), SEQ ID NO.: 2 (IIIIIGSIIGPGGEGPGGV), SEQ ID NO.: 3 (IIIIIGSIIGOGGEGPGGV), and SEQ ID NO.: 7 (IIIIIGSIIGOGAEGPGGV). These solutions were autoclaved.

Using 2 mL clear glass vials, 4 materials were prepared at concentrations of 0.1 wt%, 0.05 wt%, and 0.01 wt% in 2 mL of self-assembling peptide solution (containing a final concentration of 0.1 wt% of fibronectin) and a blank group (without self-assembling peptide solution, but containing 0.1 wt% of fibronectin). A stock solution of at a concentration of $5 \times 10^9$ erythrocytes was added to each sample to a final concentration of $1 \times 10^8$, and the mixture was homogenized by pipetting. Photographs were taken every 4 hours to compare the effects of different materials on the support for erythrocytes.

Experimental Conclusion: Among the self-assembling peptides with similar amino acid sequences, hydroxyproline and alanine had a significant effect on the cell support capacity of the scaffold solution under a liquid condition. As shown in Figure 5, at higher concentrations (0.1 wt% and 0.05 wt%), all four self-assembling peptide solutions could effectively support erythrocytes at a concentration of $1\times10^8$ erythrocytes; however, at lower concentrations (0.01 wt%), hydroxyproline, alanine, and glutamic acid in the hydrophilic domains demonstrated a significant effect on the the support capability for erythrocyte. The four self-assembling peptide solutions showed significant sedimentation at 8, 24, 32, and 48 hours, respectively. The order of support capability of the four self-assembling peptides from weakest to strongest was: SEQ ID NO.:1 (IIIIIGSIIGPGGDGPGGV) < SEQ ID NO.: 2 (IIIIIGSIIGPGGEGPGGV) < SEQ ID NO.: 3 (IIIIIGSIIGOGGEGPGGV) < SEQ ID NO.: 7 (IIIIIGSIIGOGAEGPGGV). Based on the scaffold network formed in response to fibronectin, relative to SEQ ID NO.: 1, glutamate in the hydrophilic region of SEQ ID NO.: 2, glutamic acid and hydroxyproline in the hydrophilic region of SEQ ID NO.: 3, and glutamate, hydroxyproline, and alanine in the hydrophilic region of SEQ ID NO.: 7 sequentially showed increasingly stronger enhancements in cell support capability. Hydroxyproline is abundant in the collagenous tissues of animals and can enhance the elasticity and support of the protein matrix through the hydrogen bond formed by the hydroxyl groups therein. In the present invention, the introduction of hydroxyproline can significantly enhance the stability of the β-sheet formed by self-assembly of self-assembling peptides through the enhanced hydrogen bond network between self-assembling peptides, thereby improving the stability of the self-assembling scaffold and enhancing support function for cells. Meanwhile, the β-sheet structure within the hydrophilic region enhances the hydrophobicity of the amino acid side chains without disrupting the β-turn structure of the hydrophilic region through the substitution of a single alanine for glycine, and the substitution of glutamic acid for aspartic acid. The stability of self-assembling materials is similarly increased through hydrophobic interactions, thus making the scaffold materials a stronger support for cells.

**Experiment II. Erythrocyte Support Experiment and Effect of Different Initiator Components**

Experimental Methods: 0.16 mL of erythrocyte stock solution ($9.17\times10^9$/mL) was taken and diluted to 10 mL ($9.17\times10^7$/mL) with 10 mM PBS, 4.5mL of erythrocyte dilution was added to a 10 ml of round-bottom tube and 4.5 mL of 4 mmol/L Calcein-AM solution was added, and the mixture was incubated at 37°C for 20 min and then centrifuged at 4°C, 1500r/min for 3 min. The supernatant was removed, and the pellet was resuspended in PBS, and repeated for 5 times to obtain about 1.5 mL of washed erythrocyte resuspension ($1\times10^8$/mL) for latter use.

Experimental Results: PBS buffer was added to the erythrocyte resuspension solution to dilute the erythrocytes to $1\times10^7$/mL, and the mixture was allowed to stand for 4 hours, and the result was shown in Tube A in Figure 6.

The 1 wt% of self-assembling peptide solution (SEQ ID NO.:3) obtained in Example 2 was added ro make the final peptide concentration in the system of 0.05 wt% and the final concentration of erythrocytes of $1\times10^7$/mL, and the mixture was allowed to stand for 4 hours, and the result was shown in Tube B in Figure 6.

The mixed solution of polypeptide as shown in SEQ ID NO.:3 with lysine obtained in Example 3 was added to the erythrocytes resuspension solution, i.e., the polypeptide formed a three-dimensional network scaffold material in the presence of lysine, achieving a final peptide concentration in the system of 0.05 wt.%, mixing well and standing, and the result was shown in Tube C in Figure 6.

The mixted solution of self-assembling peptide as shown in SEQ ID NO.:3 with gentamicin obtained in Example 2 was added to the erythrocytes resuspension solution, i.e., the polypeptide formed the three-dimensional network scaffold material in the presence of gentamicin, achieving a final peptide concentration in the system of 0.05 wt.%, mixing well and standing, and the result was shown in Tube D in Figure 6.

The mixted solution of self-assembling peptide as shown in SEQ ID NO.:3 with polylysine obtained in Example 2 was added to the erythrocytes resuspension solution, i.e., the self-assembling peptide formed the three-dimensional network scaffold material in the presence of polylysine, achieving a final peptide concentration in the system of 0.05 wt.%, mixing well and standing, and the result was shown in Tube E in Figure 6.

The mixted solution of self-assembling peptide as shown in SEQ ID NO.:3 with fibrinogen obtained in Example 3 was added to the erythrocytes resuspension solution, i.e., the self-assembling peptide formed the three-dimensional network scaffold material in the presence of fibrinogen, achieving a final peptide concentration in the system of 0.05 wt.%, mixing well and standing, and the result was shown in Tube F in Figure 6.

The results for the control group without the addition of self-assembling peptides or positively charged ions/group substances were shown in tube A in Figure 6. The erythrocytes concentrated at the bottom of the centrifuge tube. In Tube B, the erythrocytes underwent significant sedimentation, with a tendency to a three-dimensional distributionbut but not clearly defined. In Tubes C-E, the erythrocytes showed a stereoscopic distribution, and an increase in the height of the red portion of the centrifugal tubes was observed. In contrast to Tubes C-F, the self-assembling peptides formed a three-dimensional network scaffold material under the stimulation of different types of positively charged ions/groups, enabling the three-dimensional stereoscopic dispersion of the erythrocytes while the state of the system was still in solution. Combining with the results of Experiment I, it can be seen that the response of self-assembling peptides to positively charged ions/groups resulted in changes in the secondary structure formed by molecular assembly. These changes led to the formation of fibers with distinct morphologies and altered distribution pattern (Experiment I). Such microscopic changes explained the difference between Tubes C-F and Tubes A and B in this experiment. The erythrocytes adhered to the three-dimensional nanofibers formed in response to the self-assembling peptides, leading to their three-dimensional stereoscopic dispersion. In Tube B, where only self-assembling peptides were present, due to the overall negative charge on the cell membrane of erythrocytes, in the absence of positively charged ions/groups, self-assembling peptides could not form an effective three-dimensional network scaffold material, thus failing to provide support for the cell.

**Experiment III. Rheological Experiment and Effect of Secondary Structure of Self-Assembling Peptide and Amino Acid Sequence**

Experimental Methods: Preparation of Self-Assembling Peptide Material Samples: 2 wt% of the self-assembling peptide solution obtained in Example 2 was used, the sequences as shown in SEQ ID NO:1 (IIIIGSIIGPGGDGPGGV), SEQ ID NO:2 (IIIIGSIIGPGGEGPGGV), SEQ ID NO:3 (IIIIGSIIGOGGEGPGGV), SEQ ID NO:4 (IIIIGSIIGOGGGEGPGGV), SEQ ID NO:5 (IIIIGSIIGOGGEGPGGGV), SEQ ID NO:6 (IIIIGSIIGOGGEGPGGV), SEQ ID NO:7 (IIIIGSIIGOGAEGPGGV), SEQ ID NO:8 (IIIIIGSIIOGGAEGPGGV), SEQ ID NO:9 (IIIIIGSIIGOGGVGPGGV), and SEQ ID NO: 10 (IIIIIIGSIIGOGAEGPGGVGPGGV).

Preparation of hydrogel samples formed by self-assembling peptide solutions in response to cell complete medium: 2 wt% of stock solution of self-assembling peptides obtained in Example 2 was diluted with phosphate buffer, added by fetal bovine serum to achieve a mixture solution with a final concentration of self-assembling peptide of 0.5%, a final concentration of serum of 10 v/v% with a neutral pH.

The energy storage modulus (G') of the mixture solution was measured on a 20 mm plate using MARS 60 rheometer. In order to determine the rate of formation of three-dimensional nanomatrix, the solution was placed on a plate for testing immediately after preparation. A gap of 500$\mu$m was used, mineral oil was added to the gap to prevent dehydration of the samples, and data collection commenced immediately. Dynamic Time-Scanning experiment (DTS) was performed to monitor changes in energy storage modulus (G') with time (1 Hz frequency, 1% strain) for 2000 min.

Experimental Results: The mechanical strength of the peptide hydrogel of the present invention can effectively provide support and encapsulation effect for cells, functional molecules, drugs, etc., which facilitates the realization of three-dimensional cell culture, tissue repair, and slow release of drugs. It was demonstrated in the experiments that by mixing peptide solutions with serum, self-assembling peptides could immediately respond to the initiator substances, assembling into a hydrogel material with an energy storage modulus greater than 10. The procedure was convenient, and the gelation time was short. While comparing the different sequences (see Figures 7A-7I), it was found that the order of energy storage modulus of hydrogels was: SEQ ID NO:7 (IIIIIGSIIGOGAEGPGGV) > SEQ ID NO:3 (IIIIGSIIGOG-GEGPGV) > SEQ ID NO:2 (IIIIGSIIGPGGEGPGGV) > SEQ ID NO:1 (IIIIGSIIGPGGDGPGGV); this experimental phenomenon was consistent with the results of Experiment III, i.e., hydroxyproline can enhance the mechanical strength of the hydrogel of the present invention, thereby enhancing the support function of the scaffold material. Also comparing the results of SEQ ID NO:5 (IIIIGSIIGOGGEGPGGGV) with SEQ ID NO:6 (IIIIGSIIGOGGEGPGGV), it was found that the self-assembling peptides could still form a hydrogel structure but with weaker elasticity, when $\beta$-turn-forming amino acids were 6 amino acids (SEQ ID NO:5) and 3 amino acids (SEQ ID NO:6), and when the number of $\beta$-turn structures was increased to 3 consecutive ones (SEQ ID NO:10), or when four hydrophobic amino acids were present (SEQ ID NO:4). In Figure 7, A corresponded to sequence of SEQ ID NO:7, B corresponded to sequence of SEQ ID NO:3, C corresponded to sequence of SEQ ID NO:2, D corresponded to sequence of SEQ ID NO:1, E corresponded to sequence of SEQ ID NO:5, F corresponded to sequence of SEQ ID NO:6, G corresponded to sequence of SEQ ID NO:4, H corresponded to sequence of SEQ ID NO:10, and I corresponded to sequence of SEQ ID NO:8.

In SEQ ID NO:8, by modifying the 10th to 11th amino acids of GO in SEQ ID NO:7 to be OG, the formation condition for the first $\beta$-turn in SEQ ID NO:7 was completely disrupted, resulting in the sequence of SEQ ID NO:8; as a result, we found that SEQ ID NO:8 completely lost its self-assembly response to serum. This phenomenon further demonstrated that two consecutive $\beta$-turns are the most essential condition for the self-assembling capability of the self-assembling peptide of the present invention. While comparing SEQ ID NO:9 (IIIIIGSIIGOGGVGPGGV) with SEQ ID NO:4 (IIIIIIGSIIGOGGEGPGGV), we found that when an acidic amino acid is no longer linked to the terminus of the $\beta$-turn, the aqueous solution of the self-assembling peptide is no longer response to serum, and instead it could form a hydrogel in a neutral aqueous solution; herein we further verified that at least one acidic amino acid linked to the terminus of the $\beta$-turn in the hydrophilic region of self-assembling peptide is a prerequisite for the response of the self-assembling peptide of self-assembly material of the present invention to the initiator components.

**Example 6. Application of the Peptide Hydrogel Material of the Present Invention in the Biomedical Field**

**Experiment I. Cell Support Effect of the Self-Assembling Peptide of the Present Invention in Three-Dimensional Space after Initiation**

Cells growing on a two-dimensional surface is an artificial and unnatural way of cultivation because it greatly differs from the environment in organisms where cells can proliferate optimally. Consequently, cells harvested by conventional 2D cell culture methods are deficient in cellular structure and stimulus-response function because they are grown in an environment that is significantly different from that in organisms. However, the three-dimensional cell culture can better simulate the natural environment of cell survival in organisms, and can highly maintain the normal biochemical reactions of the cells themselves and intercellular interactions. Therefore, the cells in the three-dimensional environment have characteristic biological signals that can affect their functions such as migration, adhesion, proliferation, and gene expression, and their responses to both endogenous and exogenous stimuli are more similar to their responses *in vivo.* Specific cellular processes in tissue engineering, such as differentiation, have been shown to occur more readily in a three-dimensional environment than in a two-dimensional one. The realization of three-dimensional spatial culture of cells requires that the materials used to culture cells have excellent cell support effects and biocompatibility.

In this experiment, HepG2, a common adherent cell, was used as a cell model, and SEQ ID NO.:13 (IIIIIGOGIIGPGGEGPGGE) was used. Serum was used as a representative of a mixture of naturally occurring biomacromolecules carrying positively charged groups, and spermine as a representative of naturally occurring small molecules carrying a positive charge to initiate self-assembling peptides, and to explore the effect of three-dimensional network scaffolds formed by their assemblies on cell support. Meanwhile, in order to explore the cell support effect of the self-assembling peptide material of the present invention when applied in real cell culture and in the biomedical field, high-glucose medium, tissue fluid and serum were used as substances carrying a positively charged source to initiate the self-assembling peptide of the present invention to simulate the actual usage environment.

HepG2 cells were cultured in cell culture flasks with high-glucose medium containing 10% serum at 37°C and 5% $CO_2$ in a mildly humid environment. After culturing, cells were collected and resuspended with an appropriate amount of high-glucose medium (with 10% serum) to obtain a cell suspension with a concentration of $5 \times 10^5$ cells/mL. The prepared cell suspension and Calcein-AM/PI staining solution were mixed well in a volume ratio of 2:1, and incubated at 37°C for 15 min in a light-protected manner. The self-assembling peptide hydrogel material of the present invention, which was initiated to assemble into a three-dimensional network scaffold by various positively charged substances, was added to a glass-bottomed petri dish (see Example 3 for the method of preparaing the self-assembling peptide hydrogel), and then the cell suspension after the incubation in a light-protected manner was added and mixed well, so that the total volume of the glass-bottomed petri dish was 200, and the final concentration of the self-assembling peptide was 0.1 wt%. When preparing control samples, an equal volume of PBS buffer was used to replace the self-assembling peptide hydrogel material of the present invention used in the experimental group. Each experimental sample was then observed using an LSM 980 with Airyscan2 fast super-resolution laser confocal microscope.

Figure 8 illustrates the results of this experiment, in the control group without the self-assembling peptide hydrogel of the present invention, as shown in Figure 8A, the cells were all naturally settled at the bottom of the glass-bottomed petri dish without being supported but being a 2D planar distribution. When the self-assembling peptide of the present invention, which was initiated by the positively charged source substance, was introduced to the system, as shown in Figures 8B, 8C, 8D, and 8E, the cells within the system were all supported by the three-dimensional network scaffold formed by the self-assembling peptide assembly, presenting a stereoscopic distribution within a 3D environment.

This experimental phenomenon demonstrated that the self-assembling peptide material of the present invention can be initiated by spermine, a positively charged natural small molecule substance, to form a 3D network scaffold with good cell support effect (Figure 8B), which can effectively realize the growth and cultivation of the cells *in vitro* under the three-dimensional space, and prevent the occurrence of their sedimentation and adherence to the wall, which would otherwise lead to the growth in two-dimensional environment. Cell complete medium (Figure 8C), tissue fluid (Figure 8D) and serum (Figure 8E) are widely used comprehensive systems in cell culture and biomedical fields and carry a large number of positively charged source substances. Figures 8C, 8D, and 8E show that the peptide material of the present invention can be initiated by these three comprehensive systems to assemble into a three-dimensional network scaffold and exhibit a good cell-supporting effect. The whole initiation process is mild, without the need to add new substances other than the above three comprehensive systems, and the application is effective, convenient, and non-invasive to the cells, which demonstrates that the polypeptide material of the present invention has a great potential to be excellent in cell culture and the biomedical field.

Experiments with self-assembling peptides as shown in other sequence numbers were similar to the results of this Example and will not be repeated herein.

**Experiment II. Cytocompatibility of Self-Assembling Peptide Material of the Present Invention**

In order to demonstrate that the self-assembling peptide material of the present invention has good biocompatibility, SEQ ID NO.:13 (IIIIIGOGIIGPGGEGPGGE) was taken as an example in this experiment to measure the relative viability and proliferation of HepG2 cells over a five-day period.

The method in Experiment I for preparing a cell suspension at a concentration of $5 \times 10^5$ cells/mL was used to prepare a cell suspension at a concentration of $5 \times 10^4$ cells/mL. The cells obtained at a concentration of $5 \times 10^4$ cells/mL were inoculated into 96-well plates with each well containing about 5,000 cells. In the experimental groups, the self-assembling peptide sol prepared according to the method in Example 2 was added to achieve the concentrations of polypeptide of 0.1%, 0.3%, 0.5%, and 0.7% in the final system. In the control group, an equal volume of PBS buffer was added to replace the peptide solution, respectively. The groups were mixed well respectively and the experiment was carried out for 5 days without changing or adding fresh medium. During the experiment, 10% of the culture volume of CCK-8 kit was added every 24 hours, and after incubation for 30 minutes, the OD values of each group were measured using an enzyme labeler (parameter setting: primary wavelength of 450 nm, secondary wavelength of 630 nm), and all experiments were set up with 5 parallel groups, and the results of the experiments were shown in Figure 9A. Relative cell viability was calculated using the formula below:

$$\text{Relative cell viability } (\%) = (A_t\text{-}A_{0t})/(A_1\text{-}A_{01}) \times 100\%;$$

Wherein At is the average OD values measured after t days of culturing cells. $A_{0t}$ is the average value of blank background measured after t days. $A_1$ is the average OD value measured after 1 day of culturing cells, and $A_{01}$ is the average OD value of blank background measured after 1 day.

The cells obtained at a concentration of $5 \times 10^4$ cells/mL were also inoculated into 12-well plates with each well containing about 50,000 cells. In the experimental groups, the self-assembling peptide sol prepared according to the method in Example 2 was added to achieve the concentrations of polypeptide of 0.1%, 0.3%, 0.5%, and 0.7% in the final system. In the control group, an equal volume of PBS buffer was added to replace the peptide solution, respectively. The groups were mixed well and the experiment was carried out for 5 days without changing or adding fresh medium. Cells were collected for cell counting every 24 hr one during the experimental period, and all experiments were set up with 5 parallel groups. The results were of the experiments shown in Figure 9B.

As can be seen from the results of the relative cell viability experiments measured by the CCK-8 kit (Figure 9A), the cells in the experimental groups to which the self-assembling peptide materials of the present invention were added showed a uniform and continuous steady increase in cell viability during the 5-day experimental process, and the cells were in good condition, presenting the typical characteristics of adherent cells growing in a three-dimensional space, indicating that the cells had been supported by the three-dimensional network scaffolds assembled by the polypeptide. In contrast, the cell viability in the control group first surged and then declined precipitously, showing the typical characteristics of adherent cells growing in two-dimensional space. The two-dimensional culture would inhibit the proliferation of adherent cells due to the restriction of the growth space and the inhibition of the contact of the adherent cells, and a large number of cells died in later stages of culture. These two phenomena demonstrated that the self-assembling peptide of the present invention is not only biocompatible and non-toxic to the cells, but also enables long-term culturing of cells in a three-dimensional space *in vitro,* expands the cell growth space within the same cultivation volume, and restores the growth state of the cells in the organism.

As can be seen from the growth curves of the cells within 5 days (Figure 9B), although the total number of cells grown in the two-dimensional environment (control group) was higher than the total number of cells in each experimental groups for the first four days, the proliferation rate of the cells showed a gradually decreasing trend, and the cell state deteriorated, with a sharp drop in the total number of cells on the fifth day. In contrast, the cells in the experimental groups all grew with a relatively uniform and stable proliferation rate throughout the whole culture period, which indicates that the polypeptide material of the present invention is not only non-toxic and biocompatible to the cells, but also enables the cells to proliferate stably for a long time in a good state under the support of the network scaffold assembled with the material of the present invention. This experimental phenomenon similarly confirmed the conclusions of the experiments measuring the relative cell viability.

Furthermore, by summarizing the above two experimental phenomena, it can also be concluded that, comparing the experimental groups, the cell activity and proliferation rate will be slightly different with changes in the concentration of polypeptide materials, indicating that in the practical application of the material, the proliferation rate of cells could be regulated by adjusting the concentration of the material to better meet individual usage requirements.

HepG2 cell was used as a cell model in Experiment II, which not only confirmed the conclusion of "the polypeptide material of the present invention can effectively support cells *in vitro"* from Experiment I, but also demonstrated that the material of the present invention has good biocompatibility, and the cells can maintain high activity during the incubation period, and proliferate continuously in a good state and at a uniform rate.

Experiments with self-assembling peptides as shown in other sequence numbers were similar to the results of this Example and will not be repeated herein.

**Experiment III. Cell Culture by Using the Self-Assembling Peptide Hydrogel of the Present Invention**

The inherent property of adherent cells is that they are easy to aggregate and form spheroids when conditions prevent their adhesion to a flat surface, i.e., they grow more easily into cell spheroids in three-dimensional space. With the concentration gradient of oxygen, nutrients, and metabolic wastes inside the cell spheroid, which can simulate the multiple properties of solid tissues, the cell spheroid is an important 3D physiological model for the study of solid tumorigenesis and stem cell differentiation, and is widely used in the biomedical field. Moreover, the cell spheroid is simpler than other 3D physiological models, and the imaging analysis can be realized by common experimental means, such as optical, fluorescence, and confocal microscopy, thereby simplifying the experimental process in the research. In this experiment, common adherent cell HepG2 was cultured with the polypeptide material of the present invention that was initiated by high glucose medium, and it was verified whether the material of the present invention could achieve cell culture *in vitro* similar to that in organism by forming cell spheroid.

The method in Experiment II for preparing a cell suspension at a concentration of $5\times10^5$ cells/mL was used to prepare a cell suspension at a concentration of $1\times10^5$ cells/mL. Taking SEQ ID NO.:13 (IIIIIGOGIIGPGGEGPGGE) as an example, a cell suspension obtained at a concentration of $1\times10^5$ cells/mL was inoculated into a 24-well plate that had been pre-added with the self-assembling peptide material of the present invention (with a final concentration of the polypeptide of 0.1 wt.% in each well) so that each well contained about 100,000 cells. The medium was changed twice a week during the culture period using the "half-volume change method". In order to visualize the changes in the morphology and distribution of the cells during the culture period, the cells were stained using Calcein-AM/PI staining solution after three and seven days of culture and observed under an LSM 980 with Airyscan2 fast super-resolution laser confocal microscope, and the results were shown in Figure 10.

As shown in Figure 10A, HepG2 was not only spheroplasted but also took only 72 hours to be cultured with only 0.1 wt.% of the polypeptide material of the present invention that was initiated to assemble into a three-dimensional network scaffold by the high glucose medium, demonstrating that the self-assembling peptide material of the present invention can be used to achieve a cell culture *in vitro* that approximates to the effect of cell growth in organism, and it can effectively promote the rapid formation of spheroids by adherent cells. The size of the cell spheroids was observed to be similar after 72 hours, and the cells in them were in good condition and without apoptosis (Figure 10B). The diameter of the cell spheroid increased significantly after 7 days of culture (Figure 10C), demonstrating that the cells could proliferate in this culture system in a state of growth under three-dimensional space, i.e., the addition of the polypeptide of the present invention to the cell medium containing a positively charged source of natural substances could highly replicate the growth environment of the cells *in vivo*. Observing the cell spheroids after 7 days of culture (Figure 10D), the size of cell spheroids was similar and the cells within were in good condition. A few apoptotic cells appeared, the reason was that in the process of cell proliferation in a spherical shape, due to the concentration gradients of oxygen, nutrients, and metabolic waste gradually increased, cells at the center of the spheroid received less oxygen and nutrients while accumulating more metabolic waste, leading to apoptosis, which is a natural phenomenon.

We also observed and recorded the diameters of 50 cell spheroids in the culture system under an inverted microscope after the 5th, 10th, and 15th days of culture, respectively, and the cell spheroid diameter data collected during these three days were averaged and plotted as a bar graph (Figure 11D) to test whether HepG2 cells cultured in the polypeptide material of the present invention were able to achieve long-term proliferation *in vitro* and to photograph the characteristic cell spheroids (Figures. 11A, 11B, and 11C). As can be seen in Figure 12, under the culture system containing 0.1 wt.% of the initiated polypeptide material of the present invention, the HepG2 cells were supported and subsequently grew and proliferated in the form of cell spheroids. With the extension of the culture time, the spheroids became increasingly compact, and their diameters consistently increased by similar increments. This indicated that HepG2 cells could stably proliferate at a uniform rate over 15 days of culture, i.e., the present material can be used for long time *in vitro* three-dimensional cell culture. The relatively uniform proliferation rate of the cells also indicated that they were in good condition during the culture period and that the material of the present invention did not cause damage to the cells.

Both Experiment I and Experiment II demonstrated that the polypeptide material of the present invention has excellent cell support ability, and Experiment II also demonstrated that the polypeptide material of the present invention is biocompatible and can be used for cell culture. Experiment III demonstrated that the addition of the polypeptide of the present invention to a cell medium containing a positively charged source of natural substances can highly replicate the growth environment of cells *in vivo,* and realize a long-term three-dimensional cell culture *in vitro*.

Experiments with self-assembling peptides as shown in other sequence numbers were similar to the results of this Example and will not be repeated herein.

**Experiment IV. Evaluation of Cell Condition After Culture Using the Self-Assembling Peptide Hydrogel of the Present Invention**

In this experiment, cell spheroids cultured in Experiment III using the polypeptides as shown in SEQ ID NO.: 13 (IIIIIGOGIIGPGGEGPGGE) of the present invention at a concentration of 0.1 wt.% initiated by high-glucose medium were collected by centrifugation and digested into single cells. The applicability of the material of the present invention in cell culture and biomedical field were further evaluated by comparing the cell viability of cells cultured with the material of the present invention with that of cells cultured without the material of the present invention.

The cell spheroids in Experiment III were collected by centrifugation after 15 days of culture, and the cell spheroids were digested into single cells using trypsin, then centrifuged and collected, and an appropriate amount of high-glucose medium was added to the collected cells to prepare cell suspensions at concentrations of $5\times10^5$ and $5\times10^4$ cells/mL. Cell suspensions at a concentration of $5\times10^4$ cells/mL were inoculated into 96-well plates so that each well contained 5,000 cells. A cell suspension at a concentration of $5\times10^4$ cells/mL was prepared according to the method in Experiment VII for preparing a cell suspension at a concentration of $5\times10^5$ cells/mL and it was inoculated into a 96-well plate so that each well contained 5,000 cells, which served as a control group for this experiment. A 5-day cell viability test was carried out, during which the medium was changed daily and 5 parallel groups were set up.

As shown in Figure 12, the cell viability of the cells harvested after being cultured by the polypeptide material of the present invention initiated by the high-sugar medium had almost the same trend of change in cell viability within five days as compared with that of the control group, and the cell viability of the experimental group was also slightly better than that of the control group each day, indicating that the cells were still able to maintain their own original physiological activity after being cultured in the system containing self-assembling peptide material of the present invention assembled into a three-dimensional network fiber. This indicates that the material of the present invention will not cause damage to the physiological function of the cells in the process of cell culture, and can safely and reliably enable the cells to proliferate while maintaining their original physiological activity. And on the basis of this, it promotes growth in a state highly similar to that of *in vivo,* thereby achieving amplification on the basis without depleting the physiological functions of cells.

Experiments with self-assembling peptides as shown in other sequence numbers were similar to the results of this Example and will not be repeated herein.

**Experiment V. Cell Storage Applications**

Experimental Methods: Storage of mouse mesenchymal stem cells at 4°C was taken as an example in this experiment. The isolated mouse mesenchymal stem cells were mixed well by pipetting, 10 ml aliquots were dispensed into freezing tubes, and mixed well by pipetting so that their cell concentration was about $1\times10^6$ cells/mL. In 3D group, mouse serum, 1% of double antibody of 50% MAP+50% SFEM storage solution and the polypeptide as shown in SEQ ID NO.:20 (FLIVIGOGIIGOGGEGPGGE) were added to a final concentration of 0.05%. In 2D group, mouse serum and 1% of double antibody of 50% MAP+50% SFEM storage solution were added, followed by storage in a 4°C refrigerator.

Cell viability test:

(1) The preserved cells were thoroughly pipetted and mixed well, 500 $\mu$L of each cell suspension was taken into a 1.5 mLEP tube, PBS was added for washing, followed by centrifugation at 1,500 rpm for 10 minutes; and
(2) The supernatant was discarded, and the cells were resuspended in 150 $\mu$L of 1% PBA solution. Then, 150 $\mu$L of live/dead cell staining working solution was added, mixed thoroughly, and incubated at 37°C in a light-protected manner for 15 minutes. The samples were analyzed using a flow cytometer.

Experimental Results: To understand the changes in the survival rate of mesenchymal stem cells during the preservation period, the Calcein-AM/PI live/dead double-staining kit was used to perform double staining, and a flow cytometer was used for detection. As shown in Figure 13, the survival rate of mouse mesenchymal stem cells in the 3D storage group was significantly higher than that in the 2D storage group starting from day 3 of storage, with a statistically significant difference (79% vs. 67%). Moreover, on day 5 of storage, the 3D storage group still maintained a relatively high cell survival rate (71% vs. 43%), with an almost 30% increase in survival. This indicates that the storage system of mesenchymal stem cells by using the polypeptide of the present invention has better storage effects than that of conventional 2D storage and greatly improves cell survival rate.

Experiments with self-assembling peptides as shown in other sequence numbers were similar to the results of this Example and will not be repeated herein.

**Experiment VI. Application of the Hydrogel of the Present Invention** as a **Medical Cosmetic Dispersed Filler**

Experimental Methods: The synthesized self-assembling peptide as shown in SEQ ID NO.25 (IIIIIGOGIIGOGGGEGPGGV) was dissolved in deionized water and vortexed until fully dissolved to obtain a self-assembling peptide solution with a concentration of 1.5%, and the pH of the solution was adjusted to pH 7.4 with 0.1 M NaOH solution. Figure 14A showed, from left to right, the polypeptide solution, the precipitated poly-L-lactic acid microsphere solution, and the mixture of both. The polypeptide solution used was autoclaved, after which it was mixed with poly-L-lactic acid microspheres (particle size between 25 and 60 $\mu$ m), and finally aliquoted to obtain self-assembling peptide mixture injection containing 5% of microspheres for injection. It could be seen that the solution became opaque, and the microspheres were uniformly and stably distributed in the self-assembling peptide solution (Figure 14B). The obtained self-assembling peptide mixture injections containing microspheres were placed at room temperature for one month. It can be seen that the resulting injections were all in a uniform suspension state, none of which showed any obvious solid-liquid separation phenomenon. After the addition of 1/5 of the tissue fluid, it can be seen that a hydrogel phenomenon was occured (Figure 14C).

Similarly, using the same method and steps, 5 % of polycaprolactone (PCL) microspheres can also be supported by the peptide as shown in SEQ ID NO. 27 (FLIVIGSIIGOGAEGPGGV) with a concentration of 1.5 %. Figure 15A showed, from left to right, clear polypeptide solution of SEQ ID NO.27, the precipitated polycaprolactone (PCL) microsphere solution, and the mixture of both. The autoclaved polypeptide solution was mixed with PCL microspheres (particle size between 25 and 50 $\mu$m), making the content of PCL microspheres of 5%. It can be observed that the microspheres were uniformly and stably distributed within the self-assembling peptide solution (Figure 15B). The obtained self-assembling peptide mixture injections containing microspheres were placed at room temperature for one month. It can be seen that the resulting injections were all in a uniform suspension state, none of which showed any obvious solid-liquid separation phenomenon. After the addition of 1/5 of the tissue fluid, it can be seen that a gel phenomenon was occured (Figure 14C).

In addition, apart from SEQ ID NOs. 25 and 27, the self-assembling peptides as shown in SEQ ID NOs. 1-7 and 9-32 of the present invention could similarly support various other medical cosmetic regenerative microspheres (Table 2).

Table 2. Common Medical Cosmetology Microspheres

| Medical Cosmetology Microspheres | Support Effect | Microsphere Size |
|---|---|---|
| Polylactic acid (PLA) Microspheres | Stable Support | 20-60$\mu$m |
| Polycaprolactone (PCL) Microspheres | Stable Support | 25-50$\mu$m |
| Poly(lactic-co-glycolic acid) (PLGA) Microspheres | Stable Support | 10-150$\mu$m |
| Polyvinyl Alcohol (PVA) Microspheres | Stable Support | 10-100m |
| Poly (methyl methacrylate) (PMMA) Microspheres | Stable Support | 20-150$\mu$m |
| Polyhydroxyalkanoates (PHA)Microspheres | Stable Support | 20-60$\mu$m |
| Sodium Alginate (SA) Microspheres | Stable Support | 10-100$\mu$m |
| Poly(L-lactic acid)-poly(ethylene glycol) (PLLA-PEG) Microspheres | Stable Support | 20-45$\mu$m |

**Conclusion:** This experimental phenomenon demonstrates that the hydrogel material of the present invention, i.e., the three-dimensional network scaffold formed by self-assembly of protein-responsive self-assembling peptide in response to proteins, can effectively support the medical cosmetic microspheres, and can play a very good dispersion and supportive role for the microspheres when it is not activated. Upon initiation by proteins in tissue fluid, it can rapidly form a hydrogel, thereby functioning to provide local remodeling in the tissues. The entire process of this experiment does not require the addition of any other components and it is easy to operate.

Experiments with self-assembling peptides as shown in other sequence numbers were similar to the results of this Example and will not be repeated herein.

## Claims

1. A self-assembling peptide comprising a hydrophobic domain and a hydrophilic domain, wherein the hydrophilic domain comprises at least two consecutive $\beta$-turn regions.

2. The self-assembling peptide according to claim 1, wherein at least one $\beta$-turn region comprises or is linked to one or more acidic amino acids, preferably comprises or is linked to one acidic amino acid, at a terminus.

3. The self-assembling peptide according to claim 1, wherein each $\beta$-turn region comprises a $\beta$-turn motif formed by 3-6 amino acids, and the $\beta$-turn motif has the following structure:
$X_1X_2X_3$, $X_1X_2X_3X_4$, $X_1X_2X_3X_4X_5$, or $X_1X_2X_3X_4X_5X_6$,
wherein $X_1$, $X_2$, $X_3$, $X_4$, $X_5$, and $X_6$ are each an amino acid residue, and the $X_1$, $X_2$, $X_3$, $X_4$, $X_5$, and $X_6$ in each $\beta$-turn motif are same or different from each other, respectively.

4. The self-assembling peptide according to claim 3, wherein the β-turn motif comprises one hydroxyproline (O).

5. The self-assembling peptide according to claim 1, wherein the hydrophilic domain comprises 2-8 β-turn regions.

6. The self-assembling peptide according to claim 3, wherein the β-turn motif in the at least one β-turn region comprises or is linked to an amino acid selected from glutamic acid (E), valine (V), leucine (L), isoleucine (I), aspartic acid (D) and lysine (K) at a terminus.

7. The self-assembling peptide according to claim 3, wherein the hydrophilic domain comprises at least one β-turn motif in which $X_2$ is a hydroxyproline O, or the hydrophilic domain comprises at least one β-turn motif in which $X_2$ is a proline P.

8. The self-assembling peptide according to claim 3, wherein one or more of the $X_1$, $X_3$ and $X_4$ are glycine, and/or wherein one or two of the $X_3$ and $X_4$ are alanine (A).

9. The self-assembling peptide according to claim 1, wherein the β-turn motif comprises an amino acid sequence selected from the followings:

GPGG (SEQ ID NO.: 33), GPGA (SEQ ID NO.: 34), GPAG (SEQ ID NO.: 35), GPG, GPAA (SEQ ID NO.: 36), GPGGG (SEQ ID NO.: 37), GOGG (SEQ ID NO.: 38), GOGA (SEQ ID NO.: 39), GOAG (SEQ ID NO.: 40), GOGGA (SEQ ID NO.: 41), GOAA (SEQ ID NO.: 42), GOG, or GOGV (SEQ ID NO.: 43);
preferably, the β-turn motif has an amino acid sequence selected from the followings:
GPAGE (SEQ ID NO.: 44), GPGGE (SEQ ID NO.: 45), GOGAE (SEQ ID NO.: 46), GOGGAE (SEQ ID NO.: 47), GOGE (SEQ ID NO.: 48), GOGGE (SEQ ID NO.: 49), GPGAD (SEQ ID NO.: 50), GOGGD (SEQ ID NO.: 51), GPGGV (SEQ ID NO.: 52), GOGGV (SEQ ID NO.: 53), GPGGK (SEQ ID NO.: 54), GOGGK (SEQ ID NO.: 55), GPGAE (SEQ ID NO.: 56), GOGAD (SEQ ID NO.: 57), GPAAD (SEQ ID NO.: 58), GOAAE (SEQ ID NO.: 59), GPGGD (SEQ ID NO.:60), GPGGGV (SEQ ID NO.: 61), GPGV (SEQ ID NO.: 62), GOGGI (SEQ ID NO.: 63) or GOGVI (SEQ ID NO.: 64).

10. The self-assembling peptide according to claim 1, wherein a C-terminus of the hydrophilic domain is modified with a reagent or group selected from the group consisting of: carboxylic acid, thiol, ketoate, nitrite, phosphonate, thiophosphite, carbonate, sulfate, nitrate, vinyl sulfone, amide, alcohol, aldehyde, amine, imine, maleimide, thiol, vinyl sulfone, azide, alkyne, alkene, ester, thioester, aryl, and/or silane.

11. The self-assembling peptide according to claim 1, wherein the hydrophobic domain comprises 3-10 hydrophobic amino acids,

preferably, the hydrophobic domain comprises 3-7 hydrophobic amino acids,
preferably, the hydrophobic amino acid is selected from one or more of isoleucine (I), valine (V), leucine (L), phenylalanine (F) and alanine (A).

12. The self-assembling peptide according to claim 1, wherein N-terminus of the hydrophobic domain is modified with a reagent or group selected from the group consisting of: acetyl, alcohol, aldehyde, amine, imine, maleimide, thiol, vinyl sulfone, azide, alkyne, alkene, ester, thioester, aryl and/or silane.

13. The self-assembling peptide according to claim 1, wherein the hydrophobic domain comprises:
LLLL (SEQ ID NO.: 65), FIIII (SEQ ID NO.: 66), IIIII (SEQ ID NO.: 67), IIII (SEQ ID NO.: 68), ILILI (SEQ ID NO.: 69), FLFLF (SEQ ID NO.: 70), IVIVI (SEQ ID NO.: 71), VLFIIV (SEQ ID NO.: 72), VLIII (SEQ ID NO.: 73), IVALF (SEQ ID NO.: 74), LFIVL (SEQ ID NO.: 75), FIAIV (SEQ ID NO.: 76), FIIIV (SEQ ID NO.: 77), Ac-VLFIIV (SEQ ID NO.: 78), Ac-IVIVI (SEQ ID NO.: 79), Ac_IIIII (SEQ ID NO.: 80), IIIIII (SEQ ID NO.: 81), FLIVI (SEQ ID NO.: 82), FLIIA (SEQ ID NO.: 83), FIFIF (SEQ ID NO.: 84), IFIFI (SEQ ID NO.: 85), IAILI (SEQ ID NO.: 86) or LLLLL (SEQ ID NO.: 87).

14. The self-assembling peptide according to claim 1, further comprising a linker domain, wherein the linker domain comprises 2-8 amino acid residues, preferably 4-5 amino acid residues; and

the linker domain comprises an amino acid with a small side chain, an amino acid with a hydroxyl in the side chain, and/or a hydrophobic amino acid away from the hydrophobic domain;
the amino acid with a small side chain is selected from the group consisting of G, A, and S,

the amino acid with a hydroxyl in the side chain is selected from the group consisting of S, T and O, the hydrophobic amino acid away from the hydrophobic domain is selected from the group consisting of I, V, L, F, and A, preferably, the linker domain has an amino acid sequence selected from the followings: GPOGI (SEQ ID NO.: 88), GPOGV (SEQ ID NO.: 89), GPOGL (SEQ ID NO.: 90), GSII (SEQ ID NO.: 91), GSGII (SEQ ID NO.: 92), GSVI (SEQ ID NO.: 93), GOII (SEQ ID NO.: 94), OGII (SEQ ID NO.: 95) or GTVI (SEQ ID NO.: 96), wherein S, T, and O are interchangeable with each other,

more preferably, the linker domain has an amino acid sequence selected from the followings:
GSVL (SEQ ID NO.: 97), GSII (SEQ ID NO.: 91), GTII (SEQ ID NO.: 98), GTVI (SEQ ID NO.: 96), GOVI (SEQ ID NO.: 99), GSVI (SEQ ID NO.: 93), GSGII (SEQ ID NO.: 92), GSGVI (SEQ ID NO.: 100), GOII (SEQ ID NO.: 94), OGII (SEQ ID NO.: 95), GOGVI (SEQ ID NO.: 101) or GOGII (SEQ ID NO.: 102).

15. The self-assembling peptide according to claim 1, wherein the peptide has an amino acid sequence selected from SEQ ID NOs: 1-7 and SEQ ID NOs: 9-32.

16. A method for forming a three-dimensional network scaffold material from the self-assembling peptide according to any one of claims 1-15, wherein the method includes a step of initiating the self-assembling peptide to form a three-dimensional network scaffold material under a positively charged source initiating condition.

17. The method according to claim 16, wherein initiating the self-assembling peptide to form a three-dimensional scaffold material under a positively charged source initiating condition comprises:

initiating at a pH of less than 6; or
initiating by a positively charged source substance at a pH of 6-10.

18. The method according to claim 17, wherein the positively charged source substance is a biomacromolecule, a drug, a functional molecule, a metal ion, an amino acid, or a mixture comprising one or more endogenous or exogenous sources of the above substances, wherein

the biomacromolecule is selected from the group consisting of an organic acid, a protein, or a polysaccharide and a derivative thereof,
preferably, the organic acid is selected from the group consisting of lactic acid, tannic acid and citric acid;
preferably, the protein is a protein capable of providing a hydrogen ion under a neutral physiological condition,
preferably, the protein is a protein with an isoelectric point PI value below 7.0, preferably of 3.4-6.05,
preferably, the protein is selected from the group consisting of fibrinogen, globulin, hemoglobin, transferrin, laminin, fibronectin, and vitronectin;
preferably, the polysaccharide and derivative thereof are selected from chitin and chitosan;
the drug is selected from an antibiotic and a dopamine,
preferably, the antibiotic is selected from kanamycin and gentamicin;
the functional molecule is selected from an antioxidant and a cell proliferation promoting component,
preferably, the antioxidant is a vitamin antioxidant,
preferably, the cell proliferation promoting component is spermine or spermidine;
preferably, the metal ion is a potassium ion, a calcium ion or a magnesium ion;
preferably, the amino acid is lysine, arginine, polylysine, or polyarginine;
preferably, the positively charged source substance is serum, plasma, cell medium, tissue fluid of a plant or an animal or a mixture of one or more thereof, or animal tissue.

19. A scaffold material comprising the self-assembling peptide according to any one of claims 1-15, or prepared by the method according to any one of claims 16-18.

20. The scaffold material according to claim 19, wherein the scaffold material is a three-dimensional network scaffold material in the form of a hydrogel or a hydrogel three-dimensional network scaffold material in dried form.

21. A composition comprising the self-assembling peptide according to any one of claims 1-15 and the positively charged source substance according to claim 18.

22. The self-assembling peptide according to any one of claims 1-15, the method according to any one of claims 16-18, the scaffold material according to claim 19 or 20, or the composition according to claim 21 for use in one or more applications selected from the group consisting of: regenerative medicine and tissue regeneration; 2D and 3D cell culture and storage; dispersion and encapsulated filling of microspheres; drug delivery; wound healing; implantable material; gene therapy; stem cell therapy; and medical cosmetology.

Figure 1

Figure 2

Figure 3

Figure 4

Seq 1
IIIIIGSIIGPGGDGPGGV

Seq 2
IIIIIGSIIGPGGEGPGGV

Seq 3
IIIIIGSIIGOGGEGPGGV

Seq 7
IIIIIGSIIGOGAEGPGGV

Figure 5

Figure 6

Figure 7

Figure 8

Figure 9

Figure 10

Figure 11

Figure 12

Figure 13

Figure 14

Figure 15

**EP 4 640 694 A1**

## INTERNATIONAL SEARCH REPORT

International application No.

**PCT/CN2023/140904**

### A. CLASSIFICATION OF SUBJECT MATTER

C07K 7/08(2006.01)i; A61K 47/64(2017.01)i

According to International Patent Classification (IPC) or to both national classification and IPC

### B. FIELDS SEARCHED

Minimum documentation searched (classification system followed by classification symbols)

IPC: C07K; A61K

Documentation searched other than minimum documentation to the extent that such documents are included in the fields searched

Electronic data base consulted during the international search (name of data base and, where practicable, search terms used)

CNABS, DWPI, SIPOABS, CNKI, NCBI, ISI Web of Science: 肽, 自组装, 亲水, 疏水, 转角, 弯曲, 回折, 发夹, peptide, self assembly, hydrophilic, hydrophobic, turn, bend, reverse turn, hairpin, beta

### C. DOCUMENTS CONSIDERED TO BE RELEVANT

| Category* | Citation of document, with indication, where appropriate, of the relevant passages | Relevant to claim No. |
|---|---|---|
| X | Koki Makabe, et al. "Atomic structures of peptide self-assembly mimics" *BIOPHYSICS AND COMPUTATIONAL BIOLOGY,* Vol. 103, No. 47, 21 November 2006 (2006-11-21), 17753-17758     entire document, in particular, introduction, paragraphs 1 and 5, figure 1, and abstract | 1-22 |

☐ Further documents are listed in the continuation of Box C.     ☐ See patent family annex.

| | | |
|---|---|---|
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" document member of the same patent family |
| "P" | document published prior to the international filing date but later than the priority date claimed | |

| Date of the actual completion of the international search | Date of mailing of the international search report |
|---|---|
| **18 January 2024** | **29 January 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/140904**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201680012178
- CN 202110863352X
- CN 201810783581
- CN 201680061179
- CN 201380063880